# EUROPÄISCHE PATENTSCHRIFT

(11) **EP 1 349 846 B1**
(45) Veröffentlichungstag und Bekanntmachung des Hinweises auf die Patenterteilung: **09.02.2005**
(21) Anmeldenummer: 01985832.3
(22) Anmeldetag: 29.11.2001
(51) Int. Cl.: C07D 307/68, C07D 333/38, A61K 31/38, A61K 31/34, A61P 9/06

(54) **ARYLIERTE FURAN- UND THIOPHENCARBONSAUREAMIDE MIT KALIUM KANAL BLOCKIERENDER WIRKUNG**
ARYLATED FURANE AND THIOPHENE CARBOXYLIC ACID AMIDES WITH POTASSIUM CHANNEL BLOCKING EFFECT
AMIDES D'ACIDES FURANE ET THIOPHENE CARBOXYLIQUES ARYLES AYANT UN EFFET DE BLOCAGE DE CANAL DE POTASSIUM

(30) Priorität: 12.12.2000 DE 10061876
(43) Veröffentlichungstag der Anmeldung: 08.10.2003
(73) Patentinhaber: Aventis Pharma Deutschland GmbH, 65929 Frankfurt am Main (DE)
(72) Erfinder: PEUKERT, Stefan, 60313 Frankfurt (DE); BRENDEL, Joachim, 61118 Bad Vilbel (DE); HEMMERLE, Horst, 65812 Bad Soden (DE); KLEEMANN, Heinz-Werner, 65474 Bischofsheim (DE)
(86) Internationale Anmeldenummer: PCT/EP2001/013958
(87) Internationale Veröffentlichungsnummer: WO 2002/048131

(56) Entgegenhaltungen:
- WO-A-94/26709
- WO-A-99/07670
- DE-A- 4 412 334

## Beschreibung

Die vorliegende Erfindung betrifft Verbindungen der Formel I a und I b, 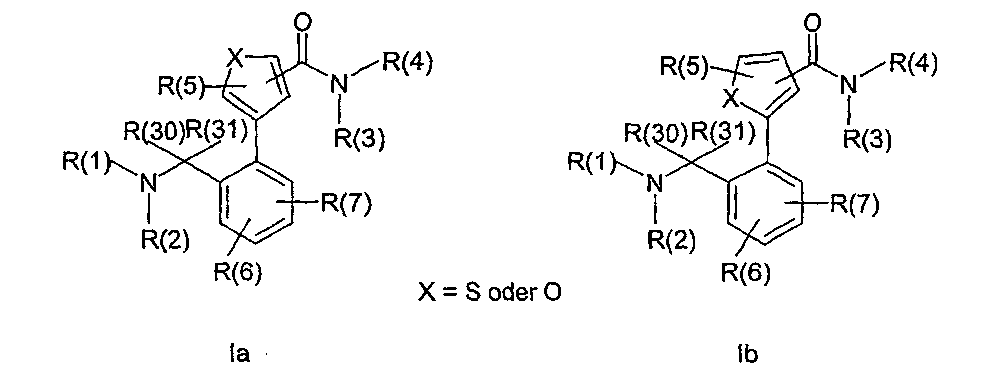 ihre Herstellung und ihre Verwendung, insbesondere in Arzneimitteln,
worin bedeuten:
- X: Sauerstoff oder Schwefel;
- R(1): C(O)OR(9), SO₂R(10), COR(11), C(O)NR(12)R(13) oder C(S)NR(12)R(13);
R(9), R(10), R(11) und R(12) unabhängig voneinander CₓH₂ₓ-R(14);
x 0, 1, 2, 3 oder 4,
wobei x nicht 0 sein kann, wenn R(14) OR(15) oder SO₂Me bedeutet;
R(14) Alkyl mit 1, 2, 3, 4, 5 oder 6 C-Atomen, Cycloalkyl mit 3, 4, 5, 6, 7, 8, 9, 10 oder 11 C-Atomen, CF₃, C₂F₅, C₃F₇, CH₂F, CHF₂, OR(15), SO₂Me, Phenyl, Naphthyl, Biphenylyl, Furyl, Thienyl oder ein N-haltiger Heteroaromat mit 1, 2, 3, 4, 5, 6, 7, 8 oder 9 C-Atomen,
wobei Phenyl, Naphthyl, Biphenylyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(15) Alkyl mit 1, 2, 3, 4 oder 5 C-Atomen, Cycloalkyl mit 3, 4, 5 oder 6 C-Atomen, CF₃ oder Phenyl, das unsubstituiert ist oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(13) Wasserstoff, Alkyl mit 1, 2, 3 oder 4 C-Atomen oder CF₃;
- R(2): Wasserstoff, Alkyl mit 1, 2, 3 oder 4 C-Atomen oder CF₃;
- R(3): CyH_{2y}-R(16);
y 0, 1, 2, 3 oder 4,
wobei y nicht 0 sein kann, wenn R(16) OR(17) oder SO₂Me bedeutet;
R(16) Alkyl mit 1, 2, 3, 4, 5 oder 6 C-Atomen, Cycloalkyl mit 3, 4, 5, 6, 7, 8, 9, 10 oder 11 C-Atomen, CF₃, C₂F₅, C₃F₇, CH₂F, CHF₂, OR(17), SO₂Me, Phenyl, Naphthyl, Furyl, Thienyl oder ein N-haltiger Heteroaromat mit 1, 2, 3, 4, 5, 6, 7, 8 oder 9 C-Atomen,
wobei Phenyl, Naphthyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino.
R(17) Wasserstoff, Alkyl mit 1, 2, 3, 4 oder 5 C-Atomen, Cycloalkyl mit 3, 4, 5 oder 6 C-Atomen, CF₃, Phenyl oder 2-, 3- oder 4-Pyridyl,
wobei Phenyl oder 2-, 3- oder 4- Pyridyl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
oder
- R(3): CHR(18)R(19);
R(18) Wasserstoff oder C_{z}H_{2z}-R(16), wobei R(16) wie oben angegeben definiert ist;
z 0, 1, 2 oder 3;
R(19) COOH, CONH₂, CONR(20)R(21), COOR(22) oder CH₂OH;
R(20) Wasserstoff, Alkyl mit 1, 2, 3, 4 oder 5 C-Atomen, CᵥH₂ᵥ-CF₃ oder C_{w}H_{2w}-Phenyl,
wobei der Phenylring unsubstituiert ist oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
v 0, 1, 2 oder 3;
w 0, 1, 2 oder 3;
R(21) Wasserstoff oder Alkyl mit 1, 2, 3, 4 oder 5 C-Atomen;
R(22) Alkyl mit 1, 2, 3, 4 oder 5 C-Atomen;
- R(4): Wasserstoff, Alkyl mit 1, 2, 3, 4, 5 oder 6 C-Atomen oder CF₃;
oder
- R(3) und R(4): gemeinsam eine Kette von 4 oder 5 Methylengruppen, von denen eine Methylengruppe durch -O-, -S-, -NH-, -N(Methyl)- oder -N(Benzyl)- ersetzt sein kann;
- R(5), R(6) und R(7): unabhängig voneinander Wasserstoff, F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl oder Methylsulfonylamino.
- R(30) und R(31): unabhängig voneinander Wasserstoff oder Alkyl mit 1, 2 oder 3 C-Atomen;
oder
- R(30) und R(31): gemeinsam Sauerstoff oder eine Kette von 2 Methylengruppen; sowie ihre pharmazeutisch akzeptablen Salze.

Bevorzugt sind Verbindungen der Formel I a und I b, in der bedeuten:
- X: Sauerstoff oder Schwefel;
- R(1): C(O)OR(9), SO₂R(10), COR(11) oder C(O)NR(12)R(13);
R(9), R(10), R(11) und R(12) unabhängig voneinander CₓH₂ₓ-R(14);
x 0, 1, 2, 3 oder 4,
wobei x nicht 0 sein kann, wenn R(14) OR(15) bedeutet
R(14) Alkyl mit 1, 2, 3, 4, C-Atomen, Cycloalkyl mit 3, 4, 5, 6, 7, 8, 9 C-Atomen, CF₃, OR(15), Phenyl, Naphthyl, Biphenylyl, Furyl, Thienyl oder ein N-haltiger Heteroaromat mit 1, 2, 3, 4, 5, 6, 7, 8 oder 9 C-Atomen,
wobei Phenyl, Naphthyl, Biphenylyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(15) Alkyl mit 1, 2, 3, 4 oder 5 C-Atomen, Cycloalkyl mit 3, 4, 5 oder 6 C-Atomen, CF₃ oder Phenyl, das unsubstituiert ist oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(13) Wasserstoff, Alkyl mit 1, 2, 3 oder 4 C-Atomen oder CF₃;
- R(2): Wasserstoff, Alkyl mit 1, 2, 3 oder 4 C-Atomen oder CF₃;
- R(3): C_{y}H_{2y}-R(16);
y 0, 1, 2, 3 oder 4,
wobei y nicht 0 sein kann, wenn R(16) OR(17) oder SO₂Me bedeutet;
R(16) Alkyl mit 1, 2, 3, 4, 5 oder 6 C-Atomen, Cycloalkyl mit 3, 4, 5, 6, 7, 8, 9, C-Atomen, CF₃, OR(17), SO₂Me, Phenyl, Naphthyl, Furyl, Thienyl oder ein N-haltiger Heteroaromat mit 1, 2, 3, 4, 5, 6, 7, 8 oder 9 C-Atomen,
wobei Phenyl, Naphthyl, Furyl, Thienyl und der N-haltige Heteroaromat unsubstituiert sind oder substituiert mit.1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(17) Wasserstoff, Alkyl mit 1, 2, 3, 4 oder 5 C-Atomen, Cycloalkyl mit 3, 4, 5 oder 6 C-Atomen, CF₃, Phenyl oder 2-, 3- oder 4-Pyridyl,
wobei Phenyl oder 2-, 3- oder 4- Pyridyl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
oder
- R(3): CHR(18)R(19);
R(18) Wasserstoff oder C_{z}H_{2z}-R(16), wobei R(16) wie oben angegeben definiert ist;
z 0, 1, 2 oder 3;
R(19) CONH₂, CONR(20)R(21), COOR(22) oder CH₂OH;
R(20) Wasserstoff, Alkyl mit 1, 2, 3, 4 oder 5 C-Atomen, CᵥH₂ᵥ-CF₃ oder C_{w}H_{2w}-Phenyl,
wobei der Phenylring unsubstituiert ist oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methytsulfonylarnino;
v 0, 1, 2 oder 3;
w 0, 1, 2 oder 3;
R(21) Wasserstoff oder Alkyl mit 1, 2, 3, 4 oder 5 C-Atomen;
R(22) Alkyl mit 1, 2, 3, 4 oder 5 C-Atomen;
- R(4): Wasserstoff, Alkyl mit 1, 2, 3, 4, 5 oder 6 C-Atomen oder CF₃;
- R(5), R(6) und R(7): unabhängig voneinander Wasserstoff, F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl oder Methylsulfonylamino;
- R(30) und R(31): unabhängig voneinander Wasserstoff oder Alkyl mit 1, 2 oder 3 C-Atomen;
oder
- R(30) und R(31): eine Kette von 2 Methylengruppen
sowie ihre pharmazeutisch akzeptablen Salze.

Besonders bevorzugt sind Verbindungen der Formel I a und I b, in der bedeuten:
- X: Sauerstoff oder Schwefel;
- R(1): C(O)OR(9), SO₂R(10), COR(11) oder C(O)NR(12)R(13);
R(9), R(10), R(11) und R(12) unabhängig voneinander CₓH₂ₓ-R(14);
x 0, 1, 2, 3 oder 4,
wobei x nicht 0 sein kann, wenn R(14) OR(15) bedeutet;
R(14) Alkyl mit 1, 2, 3, 4, 5 oder 6 C-Atomen, Cycloalkyl mit 3, 4, 5, 6, 7, 8, 9, 10 oder 11 C-Atomen, CF₃, C₂F₅, C₃F₇, CH₂F, CHF₂, OR(15), Phenyl, Naphthyl, Biphenylyl, Furyl, Thienyl oder ein N-haltiger Heteroaromat mit 1, 2, 3, 4, 5, 6, 7, 8 oder 9 C-Atomen,
wobei Phenyl, Naphthyl, Biphenylyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methytsulfonylamino;
R(15) Alkyl mit 1, 2, 3, 4 oder 5 C-Atomen, Cycloalkyl mit 3, 4, 5 oder 6 C-Atomen, CF₃ oder Phenyl, das unsubstituiert ist oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(13) Wasserstoff;
- R(2): Wasserstoff oder Alkyl mit 1, 2 oder 3 C-Atomen;
- R(3): CHR(18)R(19);
R(18) Wasserstoff oder C_{z}H_{2z}-R(16),
R(16) Alkyl mit 1, 2, 3, 4, 5 oder 6 C-Atomen, Cycloalkyl mit 3, 4, 5, 6, 7, 8, 9, C-Atomen, CF₃, OR(17), SO₂Me, Phenyl, Naphthyl, Furyl, Thienyl oder ein N-haltiger Heteroaromat mit 1, 2, 3, 4, 5, 6, 7, 8 oder 9 C-Atomen,
wobei Phenyl, Naphthyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(17) Wasserstoff, Alkyl mit 1, 2, 3, 4 oder 5 C-Atomen, Cycloalkyl mit 3, 4, 5 oder 6 C-Atomen, CF₃, Phenyl oder 2-, 3- oder 4-Pyridyl,
wobei Phenyl oder 2-, 3- oder 4- Pyridyl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
z 0, 1, 2 oder 3;
R(19) CONH₂, CONR(20)R(21), COOR(22) oder CH₂OH;
R(20) Wasserstoff, Alkyl mit 1, 2, 3, 4 oder 5 C-Atomen, CᵥH₂ᵥ-CF₃ oder C_{w}H_{2w}-Phenyl,
wobei der Phenylring unsubstituiert ist oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
v 0, 1, 2 oder 3;
w 0, 1, 2 oder 3;
R(21) Wasserstoff oder Alkyl mit 1, 2, 3, 4 oder 5 C-Atomen;
R(22) Alkyl mit 1, 2, 3, 4 oder 5 C-Atomen;
- R(4): Wasserstoff, Alkyl mit 1 oder 2 C-Atomen
- R(5), R(6) und R(7): unabhängig voneinander Wasserstoff, F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl oder Methylsulfonylamino;
- R(30) und R(31): unabhängig voneinander Wasserstoff oder Methyl;
sowie ihre pharmazeutisch akzeptablen Salze.

Ganz besonders bevorzugt sind Verbindungen der Formel I a und I b, in der bedeuten:
- X: Sauerstoff oder Schwefel;
- R(1): C(O)OR(9), SO₂R(10), COR(11) oder C(O)NR(12)R(13);
R(9), R(10), R(11) und R(12) unabhängig voneinander CₓH₂ₓ-R(14);
x 0, 1, 2, 3 oder 4,
R(14) Alkyl mit 1, 2, 3, 4, 5 oder 6 C-Atomen, Cycloalkyl mit 3, 4, 5, 6, 7, 8, 9, 10 oder 11 C-Atomen, CF₃, C₂F₅, C₃F₇, CH₂F, CHF₂, SO₂Me, Phenyl, Naphthyl, Biphenylyl, Furyl, Thienyl oder ein N-haltiger Heteroaromat mit 1, 2, 3, 4, 5, 6, 7, 8 oder 9 C-Atomen,
wobei Phenyl, Naphthyl, Biphenylyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(13) Wasserstoff;
- R(2): Wasserstoff oder Methyl;
- R(3): C_{y}H_{2y}-R(16);
y 0, 1, 2, 3 oder 4;
wobei y nicht 0 sein kann, wenn R(16) OR(17) bedeutet;
R(16) Alkyl mit 1, 2, 3, 4, 5 oder 6 C-Atomen, Cycloalkyl mit 3, 4, 5, 6, 7, 8, 9, 10 oder 11 C-Atomen, CF₃, C₂F₅, C₃F₇, CH₂F, CHF₂, OR(17), SO₂Me, Phenyl, Naphthyl, Furyl, Thienyl oder ein N-haltiger Heteroaromat mit 1, 2, 3, 4, 5, 6, 7, 8 oder 9 C-Atomen,
wobei Phenyl, Naphthyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(17) Wasserstoff, Alkyl mit 1, 2, 3, 4 oder 5 C-Atomen, Cycloalkyl mit 3, 4, 5 oder 6 C-Atomen, CF₃, Phenyl oder 2-, 3- oder 4-Pyridyl,
wobei Phenyl oder 2-, 3- oder 4- Pyridyl unsubstituiert sind oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
- R(4): Wasserstoff oder Alkyl mit 1 oder 2 C-Atomen;
- R(5), R(6) und R(7): unabhängig voneinander Wasserstoff, F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl oder Methylsulfonylamino;
- R(30) und R(31): unabhängig voneinander Wasserstoff oder Methyl;
sowie ihre pharmazeutisch akzeptablen Salze.

Besonders speziell bevorzugt sind Verbindungen der Formel I a oder I b, in der bedeuten:
- R(1): C(O)OR(9), SO₂R(10), COR(11) oder C(O)NR(12)R(13);
R(9), R(10), R(11) und R(12) unabhängig voneinander CₓH₂ₓ-R(14);
x 0, 1, 2 oder 3;
R(14) Alkyl mit 1, 2, 3 oder 4 C-Atomen, Cycloalkyl mit 3, 4, 5, 6, 7, 8 oder 9 C-Atomen, CF₃, Phenyl oder Pyridyl,
wobei Phenyl und Pyridyl unsubstituiert sind oder substituiert mit 1 oder 2 Substituenten ausgewählt aus der Gruppe bestehend aus F, Cl, Br, I, CF₃, OCF₃, OH, Alkyl mit 1, 2, 3, Alkoxy mit 1 oder 2 C-Atomen;
R(13) Wasserstoff:
- R(2): Wasserstoff;
- R(3): C_{y}H_{2y}-R(16);
y 0, 1 oder 2;
R(16) Alkyl mit 1, 2 oder 3 C-Atomen, Cycloalkyl mit 5 oder 6 C-Atomen, CF₃, Phenyl oder Pyridyl,
wobei Phenyl und Pyridyl unsubstituiert sind oder substituiert mit 1 oder 2 Substituenten ausgewählt aus der Gruppe bestehend aus F, Cl, CF₃, OCF₃, Alkyl mit 1, 2 oder 3 C-Atomen oder Alkoxy mit 1 oder 2 C-Atomen;
- R(4): Wasserstoff;
- R(5), R(6) und R(7): unabhängig voneinander F, Cl, CF₃, CN, COOMe, CONH₂, COMe, NH₂, OH, Alkyl mit 1, 2 oder 3 C-Atomen, Alkoxy mit 1 oder 2 C-Atomen;
- R(30) und R(31): unabhängig voneinander Wasserstoff oder Methyl;
sowie ihre pharmazeutisch akzeptablen Salze.

Insbesondere speziell bevorzugt sind. Verbindungen der Formel I a und I b, in der bedeuten:
- X: Sauerstoff oder Schwefel;
- R(1): C(O)OR(9) oder COR(11);
R(9) und R(11) unabhängig voneinander CₓH₂ₓ-R(14);
x 0, 1, 2 oder 3;
R(14) Cycloalkyl mit 5 oder 6 C-Atomen oder Phenyl,
wobei Phenyl unsubstituiert sind oder substituiert mit 1 oder 2 Substituenten ausgewählt aus der Gruppe bestehend aus F, Cl, Br, I, CF₃, OCF₃, OH, Alkyl mit 1, 2, 3 oder Alkoxy mit 1 oder 2 C-Atomen;
- R(2): Wasserstoff;
- R(3): C_{y}H_{2y}-R(16);
y 0, 1 oder 2;
R(16) Alkyl mit 1, 2 oder 3 C-Atomen, Cycloalkyl mit 5 oder 6 C-Atomen, Phenyl oder Pyridyl,
wobei Phenyl und Pyridyl unsubstituiert sind oder substituiert mit 1, 2 oder 3 Substituenten ausgewählt aus der Gruppe bestehend aus F, Cl, CF₃, Alkyl mit 1, 2 oder 3 C-Atomen oder Alkoxy mit 1 oder 2 C-Atomen;
- R(4): Wasserstoff;
- R(5), R(6) und R(7): unabhängig voneinander Wasserstoff, F, Cl, Alkyl mit 1, 2 oder 3 C-Atomen oder Alkoxy mit 1, 2 C-Atomen;
- R(30) und R(31): Wasserstoff;
sowie ihre pharmazeutisch akzeptablen Salze.

Die erfindungsgemässen Verbindungen sind bisher nicht bekannt.
Sie wirken auf den sogenannten Kv1.5-Kalium-Kanal und inhibieren einen als "ultrarapidty activating delayed rectifier" bezeichneten Kaliumstrom im humanen Herzvorhof. Die Verbindungen sind deshalb ganz besonders geeignet als neuartige antiarrhythmische Wirkstoffe, insbesondere zur Behandlung und Prophylaxe von Vorhof-Arrhythmien, z.B. Vorhof-Flimmem (atriale Fibrillation, AF) oder Vorhof-Flattern (atriales Flattern).

Vorhof-Flimmern (AF) und Vorhof-Flattern sind die häufigsten anhaltenden Herzarrhythmien. Das Auftreten erhöht sich mit zunehmenden Alter und führt häufig zu fatalen Folgeerscheinungen, wie zum Beispiel Gehimschlag. AF betrifft ca. 1 Millionen Amerikaner jährlich und führt zu mehr als 80.000 Schlaganfällen jedes Jahr in den USA. Die zur Zeit gebräuchlichen Antiarrhythmika der Klasse I und III reduzieren die Wiederauftrittsrate von AF, finden aber wegen ihrer potentiellen proarrhythmischen Nebenwirkungen nur eingeschränkte Anwendung. Deshalb besteht eine hohe medizinische Notwendigkeit für die Entwicklung besserer Medikamente zur Behandlung atrialer Arrhythmien (S. Nattel, Am. Heart J. 130, 1995, 1094 - 1106; "Newer developments in the management of atrial fibrillation").

Es wurde gezeigt, dass den meisten supraventrikulären Arrhythmien sogenannte "Reentry" Erregungswellen unterliegen. Solche Reentries treten dann auf, wenn das Herzgewebe eine langsame Leitfähigkeit und gleichzeitig sehr kurze Refraktärperioden besitzt. Das Erhöhen der myokardialen Refraktärzeit durch Verlängerung des Aktionspotentials ist ein anerkannter Mechanismus, um Arrhythmien zu beenden bzw. deren Entstehen zu verhindern (T.J. Colatsky et al, Drug Dev. Res. 19, 1990, 129 - 140; "Potassium channels as targets for antiarrhythmic drug action"). Die Länge des Aktionspotentials wird im wesentlichen bestimmt durch das Ausmaß repolarisierender K⁺-Ströme, die über verschiedene K⁺-Kanäle aus der Zelle herausfließen. Eine besonders große Bedeutung wird hierbei dem sogenannten "delayed rectifier" I_{K} zugeschrieben, der aus 3 verschiedenen Komponenten besteht: IKᵣ, IKₛ und lKᵤᵣ.

Die meisten bekannten Klasse III- Antiarrhythmika (z.B. Dofetilide, E4031 und d-Sotalol) blockieren überwiegend oder ausschließlich den schnell aktivierenden Kaliumkanal IKᵣ, der sich sowohl in Zellen des menschlichen Ventrikel als auch im Vorhof nachweisen lässt. Es hat sich jedoch gezeigt, dass diese Verbindungen bei geringen oder normalen Herzfrequenzen ein erhöhtes proarrhythmisches Risiko aufweisen, wobei insbesondere Arrhythmien, die als "Torsades de pointes" bezeichnet werden, beobachtet wurden (D. M. Roden, Am. J. Cardiol. 72, 1993, 44B-49B; "Current status of class III antiarrhythmic drug therapy"). Neben diesem hohen, zum Teil tödlichen Risiko bei niedriger Frequenz, wurde für die I_{Kr}-Blocker ein Nachlassen der Wirksamkeit unter den Bedingungen von Tachykardie, in der die Wirkung gerade benötigt wird, festgestellt ("negative use-dependence").

Während einige dieser Nachteile durch Blocker der langsam aktivierenden Komponente (IK_{S}) möglicherweise überwunden werden können, wurde deren Wirksamkeit bisher nicht bewiesen, da keine klinischen Untersuchungen mit IKₛ-Kanalblockem bekannt sind.
Die "besonders schnell" aktivierende und sehr langsam inaktivierende Komponente des delayed Rectifier IKᵤᵣ (=ultra-rapidly activating delayed rectifier), die dem Kv1.5-Kanal entspricht, spielt eine besonders große Rolle für die Repolarisationsdauer im menschlichen Vorhof. Eine Inhibierung des IKᵤᵣ-Kaliumauswärtsstroms stellt somit im Vergleich zur Inhibierung von IKᵣ bzw. IKₛ eine besonders effektive Methode zur Verlängerung des atrialen Aktionspotentials und damit zur Beendigung bzw. Verhinderung von atrialen Arrhythmien dar. Mathematische Modelle des menschlichen Aktionspotentials legen nahe, dass der positive Effekt einer Blockade des IKᵤᵣ gerade unter den pathologischen Bedingungen einer chronischen atrialen Fibrillation besonders ausgeprägt sein sollte (M. Courtemanche, R. J. Ramirez, S. Nattel, Cardiovascular Research 1999, 42, 477 - 489: "Ionic targets for drug therapy and atrial fibrillation-induced electrical remodeling: insights from a mathematical model).

Im Gegensatz zu IKᵣ und IKₛ, die auch im menschlichen Ventrikel vorkommen, spielt der IKᵤᵣ zwar eine bedeutende Rolle im menschlichen Vorhof, jedoch nicht im Ventrikel. Aus diesem Grunde ist bei Inhibierung des IKᵤᵣ-Stroms im Gegensatz zur Blockade von IKᵣ oder IKₛ das Risiko einer proarrhythmischen Wirkung auf den Ventrikel von vornherein ausgeschlossen. (Z. Wang et al, Circ. Res. 73, 1993, 1061 - 1076: "Sustained Depolarisation-Induced Outward Current in Human Atrial Myocytes"; G.-R. Li et al, Circ. Res. 78, 1996, 689 - 696: "Evidence for Two Components of Delayed Rectifier K⁺-Current in Human Ventricular Myocytes"; G. J. Amos et al, J. Physiol. 491, 1996, 31 - 50: "Differences between outward currents of human atrial and subepicardial ventricular myocytes").

Antiarrhythmika, die über eine selektive Blockade des IKᵤᵣ-Stroms bzw. Kv1.5-Kanals wirken, sind auf dem Markt bisher jedoch nicht verfügbar. Für zahlreiche pharmazeutische Wirkstoffe (z.B. Tedisamil, Bupivacaine oder Sertindole) wurde zwar eine blockierende Wirkung auf den Kv1.5-Kanal beschrieben, doch stellt die Kv1.5-Blockade hier jeweils nur eine Nebenwirkung neben anderen Hauptwirkungen der Substanzen dar.

In WO 98 04 521 und WO 99 37 607 werden Aminoindane und Aminotetrahydronaphtaline als Kaliumkanalblocker beansprucht, die den Kv1.5-Kanal blockieren. Ebenfalls als Kv1.5-Blocker werden strukturell verwandte Aminochromane in WO 00 12 077 beansprucht. In der Anmeldung WO 99 62 891 werden Thiazolidinone beansprucht die ebenfalls den Kaliumkanal blockieren. In den Anmeldungen WO 98 18 475 und WO 98 18 476 wird die Verwendung verschiedener Pyridazinone und Phosphinoxide als Antiarrhythmika beansprucht, die über eine Blockade des IKᵤᵣ wirken sollen. Die gleichen Verbindungen wurden ursprünglich jedoch auch als Immunsuppressiva beschrieben (WO 96 25 936). Alle in oben genannten Anmeldungen beschriebenen Verbindungen sind strukturell völlig andersartig als die erfindungsgemäßen Verbindungen dieser Anmeldung. Von allen in den oben genannten Anmeldungen beanspruchten Verbindungen sind uns keine klinischen Daten bekannt.

Es wurde nun überraschenderweise gefunden, dass die hier beschriebenen arylierten Furan- und Thiophencarbonsäureamide potente Blocker des humanen Kv1.5-Kanals sind. Sie können deshalb verwendet werden als neuartige Antiarrhythmika mit besonders vorteilhaftem Sicherheitsprofil. Insbesondere eignen sich die Verbindungen zur Behandlung supraventrikulärer Arrhythmien, z. B. Vorhof-Flimmern oder Vorhof-Flattern.

Die Verbindungen können eingesetzt werden zur Terminierung von bestehendem Vorhof-Flimmern oder -Flattern zur Wiedererlangung des Sinus-Rhythmus (Kardioversion). Darüber hinaus reduzieren die Substanzen die Anfälligkeit zur Entstehung neuer Flimmer-Ereignisse (Erhalt des Sinus-Rhythmus, Prophylaxe).

Die erfindungsgemässen Verbindungen sind bisher nicht bekannt.

Ist von Verbindungen der Formel I in dieser Erfindung die Rede sind dabei stets Verbindungen der Formel I a und I b gemeint-Nach der Erfindung können Alkylreste und Alkylenreste geradkettig oder verzweigt sein. Dies gilt auch für die Alkylenreste der Formeln CₓH₂ₓ, C_{y}H_{2y}, C_{z}H_{2z}, CᵥH₂ᵥ und C_{w}H_{2w}. Alkylreste und Alkylenreste können auch geradkettig oder verzweigt sein, wenn sie substituiert sind oder in anderen Resten enthalten sind, z. B. in einem Alkoxyrest oder in einem fluorierten Alkylrest. Beispiele für Alkylreste sind Methyl, Ethyl, n-Propyl, Isopropyl, n-Butyl, Isobutyl, sec-Butyl, tert-Butyl, n-Pentyl, Isopentyl, Neopentyl, n-Hexyl, 3,3-Dimethylbutyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl. Die von diesen Resten abgeleiteten zweiwertigen Reste, z. B. Methylen, 1,1-Ethylen, 1,2-Ethylen, 1,1-Propylen, 1,2-Propylen, 2,2-Propylen, 1,3-Propylen, 1,1-Butylen, 1,4- Butylen, 1,5- Pentylen, 2,2-Dimethyl-1,3-propylen, 1,6- Hexylen, usw. sind Beispiele für Alkylenreste.

Cycloalkylreste können ebenfalls verzweigt sein. Beispiele für Cycloalkylreste mit 3 bis 11 C-Atomen sind Cyclopropyl, Cyclobutyl, 1-Methylcyclopropyl, 2-Methylcyclopropyl, 2-Methylcyclobutyl, 3-Methylcyclobutyl, Cyclopentyl, Cyclohexyl, 2-Methylcyclohexyl, 3-Methylcyclohexyl, 4-Methylcyclohexyl, Menthyl, Cycloheptyl, Cyclooctyl usw.

Als N-haltige Heteroaromaten mit 1, 2, 3, 4, 5, 6, 7, 8 oder 9 C-Atomen gelten insbesondere 1-, 2- oder 3- Pyrrolyl, 1-, 2-, 4- oder 5-lmidazolyl, 1-, 3-, 4- oder 5-Pyrazolyl, 1,2,3-Triazol-1-, -4- oder 5-yl, 1,2,4-Triazol-1-, -3- oder -5-yl, 1- oder 5-Tetrazolyl, 2-, 4- oder 5-Oxazolyl, 3-, 4- oder 5-Isoxazolyl, 1,2,3-Oxadiazol-4- oder 5-yl, 1,2,4-Oxadiazol-3- oder 5-yl, 1,3,4-Oxadiazol-2-yl oder -5-yl, 2-, 4- oder 5-Thiazolyl, 3-, 4- oder 5-lsothiazolyl, 1,3,4-Thiadiazol-2- oder -5-yl, 1,2,4-Thiadiazol-3- oder -5-yl, 1,2,3-Thiadiazol-4- oder 5-yl, 2-, 3- oder 4-Pyridyl, 2-, 4-, 5- oder 6-Pyrimidinyl, 3- oder 4-Pyridazinyl, Pyrazinyl, 1-, 2-, 3-, 4-, 5-, 6- oder 7-Indolyl, 1-, 2-, 4- oder 5-Benzimidazotyl, 1-, 3-, 4-, 5-, 6- oder 7-lndazolyl, 2-, 3-, 4-, 5-, 6-, 7- oder 8-Chinolyl, 1-, 3-, 4-, 5-, 6-, 7- oder 8-lsochinolyl, 2-, 4-, 5-, 6-, 7- oder 8-Chinazolinyl, 3-, 4-, 5-, 6-, 7- oder 8-Cinnolinyl, 2-, 3-, 5-, 6-, 7- oder 8-Chinoxalinyl, 1-, 4-, 5-, 6-, 7- oder 8-Phthalazinyl. Umfasst sind weiterhin die entsprechenden N-Oxide dieser Verbindungen, also z.B. 1-Oxy-2-, 3- oder 4-pyridyl.

Besonders bevorzugt sind die N-haltigen Heterocyclen Pyrrolyl, Imidazolyl, Chinolyl, Pyrazolyl, Pyridyl, Pyrazinyl, Pyrimidinyl und Pyridazinyl.

Pyridyl steht sowohl für 2-, 3- als auch 4-Pyridyl. Thienyl steht sowohl für 2- als auch 3-Thienyl. Furyl steht sowohl für 2- als auch 3-Furyl.

Monosubstituierte Phenylreste können in der 2-, der 3- oder der 4-Position substituiert sein, disubstituierte in der 2,3-, 2,4-, 2,5-, 2,6-, 3,4- oder 3,5-Position, trisubstituierte in der 2,3,4-, 2,3,5-, 2,3,6-, 2,4,5-, 2,4,6- oder 3,4,5-Position. Entsprechendes gilt sinngemäß analog auch für die N-haltigen Heteroaromaten, den Thiophen- oder die Furylrest.

Bei Di- bzw. Trisubstitution eines Restes können die Substituenten gleich oder verschieden sein.

Wenn R(3) und R(4) gemeinsam eine Kette von 4 oder 5 Methylengruppen bedeuten, von denen eine Methylengruppe durch -O-, -S-, -NH- etc. ersetzt sein kann, dann bilden diese Reste zusammen mit dem Stickstoffatom der Formel I einen 5 oder 6-gliedrigen Stickstoffheterocyclus, wie z.B. Pyrrolidin, Piperidin, Morpholin, Thiomorpholin etc.

Enthalten die Verbindungen der Formel I eine oder mehrere saure oder basische Gruppen bzw, einen oder mehrere basische Heterocyclen, so gehören auch die entsprechenden physiologisch oder toxikologisch verträglichen Salze zur Erfindung, insbesondere die pharmazeutisch verwendbaren Salze. So können die Verbindungen der Formel I, die saure Gruppen, z. B. eine oder mehrere COOH-Gruppen, tragen, beispielsweise als Alkalimetallsalze, vorzugsweise Natrium- oder Kaliumsalze, oder als Erdalkalimetallsalze, z. B. Calcium- oder Magnesiumsalze, oder als Ammoniumsalze, z. B. als Salze mit Ammoniak oder organischen Aminen oder Aminosäuren, verwendet werden. Verbindungen der Formel I, die eine oder mehrere basische, d. h. protonierbare, Gruppen tragen oder einen oder mehrere basische heterocyclische Ringe enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw. Enthalten die Verbindungen der Formel I gleichzeitig saure und basische Gruppen im Molekül, so gehören neben den geschilderten Salzformen auch innere Salze, sogenannte Betaine, zu der Erfindung. Salze können aus den Verbindungen der Formel I nach üblichen Verfahren erhalten werden, beispielsweise durch Vereinigung mit einer Säure bzw. Base in einem Lösungs- oder Dispergiermittel oder auch durch Anionenaustausch aus anderen Salzen.

Die Verbindungen der Formel I können bei entsprechender Substitution in stereoisomeren Formen vorliegen. Enthalten die Verbindungen der Formel I ein oder mehrere Asymmetriezentren, so können diese unabhängig voneinander die S-Konfiguration oder die R-Konfiguration aufweisen. Zur Erfindung gehören alle möglichen Stereoisomeren, z. B. Enantiomere oder Diastereomere, und Mischungen von zwei oder mehr stereoisomeren Formen, z. B. Enantiomeren und/oder Diastereomeren, in beliebigen Verhältnissen. Enantiomere z. B. gehören also in enantiomerenreiner Form, sowohl als links- als auch als rechtsdrehende Antipoden, und auch in Form von Mischungen der beiden Enantiomeren in unterschiedlichen Verhältnissen oder in Form von Racematen zu der Erfindung. Die Herstellung von einzelnen Stereoisomeren kann gewünschtenfalls durch Auftrennung eines Gemisches nach üblichen Methoden oder z. B. durch stereoselektive Synthese erfolgen. Bei Vorliegen von beweglichen Wasserstoffatomen umfasst die vorliegende Erfindung auch alle tautomeren Formen der Verbindungen der Formel I.

Die Verbindungen der Formel I a und I b sind durch unterschiedliche chemische Verfahren herstellbar, die ebenfalls zu der vorliegenden Erfindung gehören. Einige typische Wege sind in den unten als Schemata 1 bis 4 bezeichneten Reaktionssequenzen skizziert. X und die Reste R(1 ) bis R(7), R(30) und R (31) sind jeweils wie oben angegeben definiert, sofern nachfolgend nicht etwas anderes angegeben ist.

So erhält man beispielsweise eine Verbindung der Formel I b gemäß Schema 1 (Methode A) oder Schema 2 (Methode B). Auf analoge Weise lassen sich Verbindungen der Formel I a unter Verwendung der entsprechenden Halogenide III a (Struktur siehe Schema 4) herstellen.

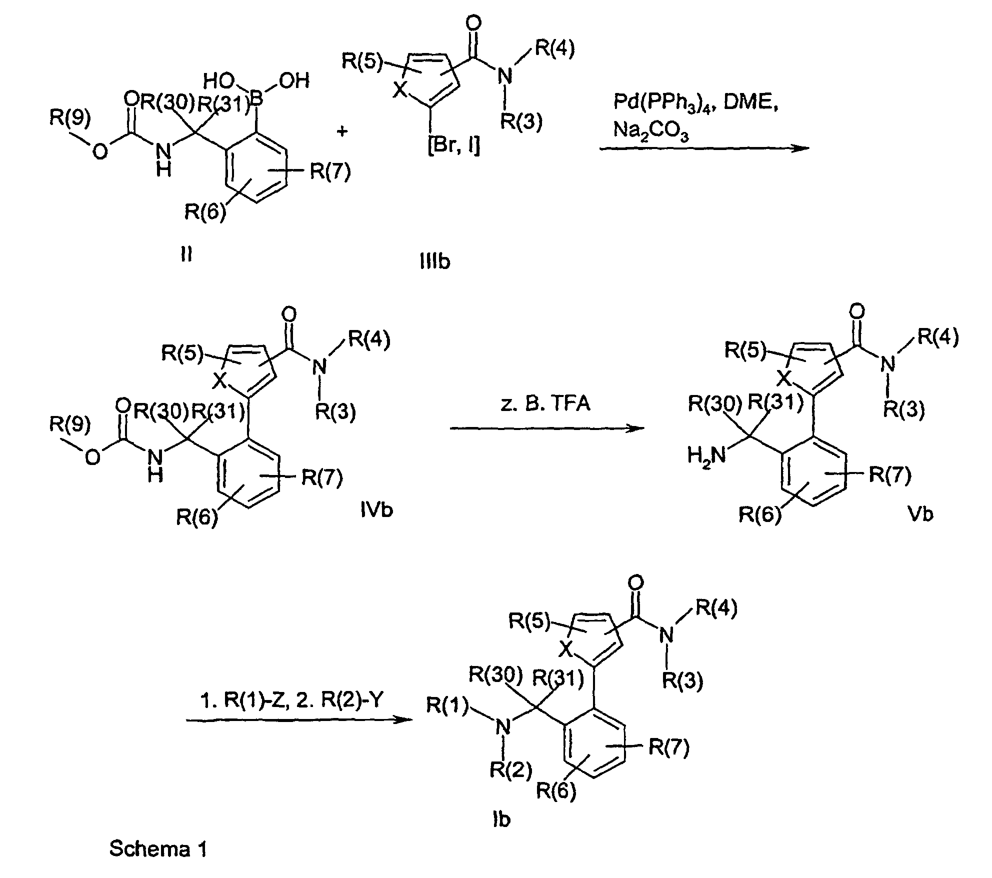

Arylierte Furan- und Thiophencarbonsäureamide der Formel IV b können durch Palladium-katalysierte Suzuki-Kupplung (die z. B. in Gegenwart von Pd[(PPh₃)]₄ als Katalysator, Natriumcarbonat als Base und 1,2-Dimethoxyethan als Lösungsmittel durchgeführt werden kann) eines aromatischen Halogenids der Formel III b mit einer aromatischen Boronsäure der Formel II dargestellt werden. Wenn R(9) für einen leicht abspaltbaren Rest, wie z. B. tert.-Butyl oder Benzyl steht, können Verbindungen Formel V b erhalten werden, die dann durch Umsetzung mit Reagenzien R(1)-Z und/oder R(2)-Y in Verbindungen der Formel I überführt werden können. Die Umsetzungen der Verbindungen der Formel V b mit Verbindungen der Formel R(1)-Z entsprechen der bekannten Umwandlung eines Amins in ein Carbonsäureamid-, Sulfonsäureamid-, Carbamat-, Harnstoff- oder Thiohamstoffderivat. Der Rest Z steht hierbei für eine geeignete nucleofuge Abgangsgruppe, wie z.B. F, Cl, Br, Imidazol, O-Succinimid etc.

Zur Herstellung von Verbindungen der Formel I b in denen R(1 ) C(O)OR(9) bedeutet, also Carbamaten, werden z.B. Verbindungen der Formel R(1)-Z verwendet, bei denen Z für Chlor oder O-Succinimid steht, also Chloroformiate oder Succinimidocarbonate.

Zur Herstellung von Verbindungen der Formel I b in denen R(1) SO₂R(10) bedeutet, also Sulfonamiden, werden in der Regel Verbindungen der Formel R(1)-Z verwendet, bei denen Z für Chlor steht, also Sulfonsäurechloride.

Zur Herstellung von Verbindungen der Formel I b in denen R(1 ) COR(11) bedeutet, also Carbonsäureamiden, werden z.B. Verbindungen der Formel R(1)-Z verwendet, bei denen Z für Chlor, Imidazol oder Acetoxy steht, also Carbonsäurechloride, Carbonsäureimidazolide oder gemischte Anhydride. Es können aber auch die freien Säuren der Formel R(1)-OH in Gegenwart geeigneter Kondensationsmittel wie Carbodiimiden oder TFFH verwendet werden.

Zur Herstellung von Verbindungen der Formel I b, in denen R(1) CONR(12)R(13) oder C(S)NR(12)R(13) bedeutet, also Harnstoffen oder Thioharnstoffen, können anstelle der Verbindungen der Formel R(1)-Z auch Verbindungen der Formel R(12)N(=C=O), bzw. R(12)N(=C=S), also Isocyanate oder Thioisocyanate verwendet werden.

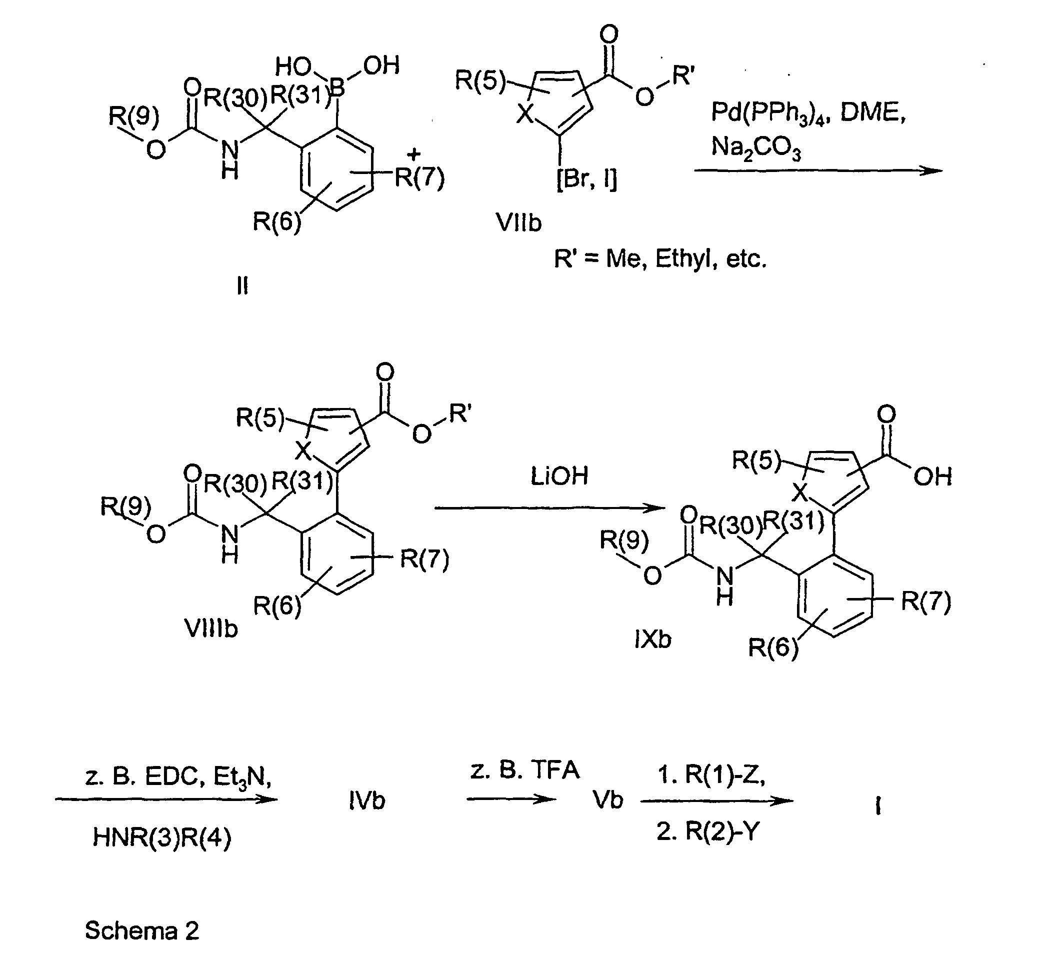

Arylierte Furan- und Thiophencarbonsäureamide der Formel VIII b können durch Palladium-katalysierte Suzuki-Kupplung eines aromatischen Bromids oder lodids der Formel VII b mit einer aromatischen Boronsäure der Formel II dargestellt werden. Hydrolyse des Esters mit LiOH ergibt die freien Säuren der Formel IX b die durch Kupplung mit Aminen NHR(3)R(4) in die Bisaryle der Formel IV b überführt werden können. Wie in Schema 1 beschrieben liefert Abspaltung der labilen Gruppe R(9) Verbindungen der Formel V b, die weiter in Verbindungen der Formel I b umgesetzt werden können. Auf analoge Weise lassen sich Verbindungen I a durch Verwendung der entsprechenden Halogenide VII a (Struktur in Schema 4) herstellen.

Die oben genannten Umsetzungen der Verbindungen der Formeln IX b mit Aminen der Formel HNR(3)R(4) entsprechen der bekannten Umwandlung einer Carbonsäure zu einem Carbonsäureamid. Zur Durchführung dieser Reaktionen sind in der Literatur zahlreiche Methoden beschrieben worden. Besonders vorteilhaft können sie durch Aktivierung der Carbonsäure, z.B. mit Dicyclohexylcarbodiimid (DCC) oder N-Ethyl-N'-(3-dimethylaminopropyl)carbodiimid-hydrochlorid (EDC) gegebenenfalls unter Zusatz von Hydroxybenzotriazol (HOBt) oder Dimethylaminopyridin (DMAP). Es können aber auch zunächst nach bekannten Methoden reaktive Säurederivate synthetisiert werden, z.B. Säurechloride durch Umsetzung der Carbonsäuren der Formel IX oder mit anorganischen Säurehalogeniden, wie z.B. SOCl₂, oder Säureimidazolide durch Umsetzung mit Carbonyldiimidazol, die dann anschließend, gegebenenfalls unter Zusatz einer Hilfsbase, mit den Aminen der Formel HNR(3)R(4) umgesetzt werden.

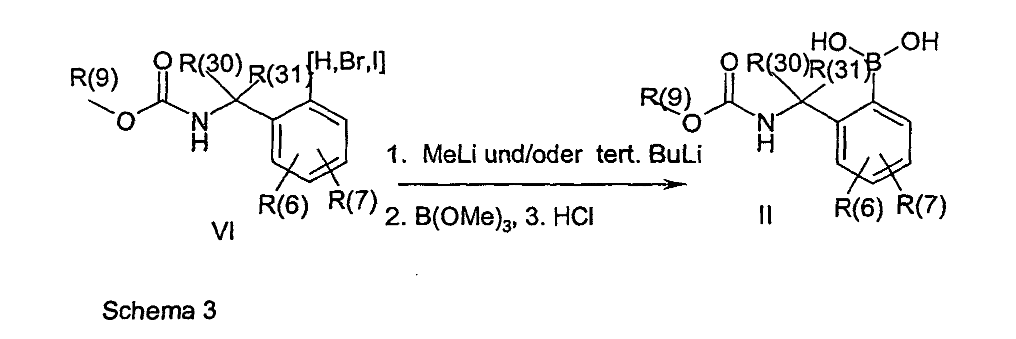

Die in Methode A und B benötigten aromatischen Boronsäuren der Formel II können aus den Aromaten oder aromatischen Halogeniden der Formel VI durch Ortholithiierung bzw. Halogen-Metallaustausch gefolgt von Umsetzung mit Borsäuretrimethylester (oder anderer Borsäuretriester) und anschliessende saure Hydrolyse synthetisiert werden.

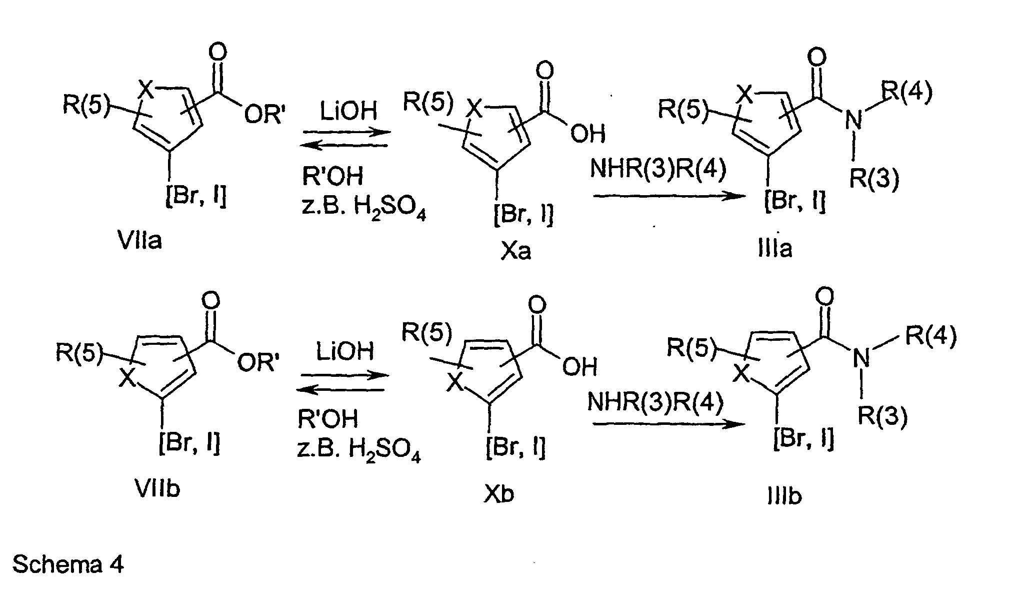

Die in Methode B eingesetzten Halogenide der Formel VII a und VII b sind nach literaturbekannten Vorschriften synthetisierbar bzw. leicht durch gängige Veresterungsmethoden aus den literaturbekannten Säuren der Formel X erhältlich. Die in Methode A eingesetzten aromatischen ortho-Halogenamide der Formel III a und III b sind gemäss Schema 4 aus den Estern der Formel VII a und VII b nach Hydrolyse zu den Säuren X a und X b durch Kupplung mit Aminen NHR(3)R(4) erhältlich. Die Knüpfung der Amidbindung kann auf den oben für die Umsetzung von Verbindungen der Formel IX b nach IV b beschriebenen Wegen erfolgen.

Bei allen Verfahrensweisen kann es angebracht sein, bei bestimmten Reaktionsschritten funktionelle Gruppen im Molekül zeitweilig zu schützen. Solche Schutzgruppentechniken sind dem Fachmann geläufig. Die Auswahl einer Schutzgruppe für in Betracht kommende funktionelle Gruppen und die Verfahren zu ihrer Einführung und Abspaltung sind in der Literatur beschrieben und können gegebenenfalls ohne Schwierigkeiten dem Einzelfall angepaßt werden.

Die erfindungsgemäßen Verbindungen der Formel I a und I b und ihre physiologisch verträglichen Salze können am Tier, bevorzugt am Säugetier, und insbesondere am Menschen als Arzneimittel für sich allein, in Mischungen untereinander oder in Form von pharmazeutischen Zubereitungen verwendet werden. Gegenstand der vorliegenden Erfindung sind auch die Verbindungen der Formel I a und I b und ihre physiologisch verträglichen Salze zur Anwendung als Arzneimittel, ihre Verwendung in der Therapie und Prophylaxe der genannten Krankheitsbilder und ihre Verwendung zur Herstellung von Medikamenten dafür und von Medikamenten mit K⁺-Kanalblockierender Wirkung. Weiterhin sind Gegenstand der vorliegenden Erfindung pharmazeutische Zubereitungen, die als aktiven Bestandteil eine wirksame Dosis mindestens einer Verbindung der Formel I a und I b und/oder eines physiologisch verträglichen Salzes davon neben üblichen, pharmazeutisch einwandfreien Trägerund Hilfsstoffen enthalten. Die pharmazeutischen Zubereitungen enthalten normalerweise 0,1 bis 90 Gewichtsprozent der Verbindungen der Formel I a und I b und/oder ihrer physiologisch verträglichen Salze. Die Herstellung der pharmazeutischen Zubereitungen kann in an sich bekannter Weise erfolgen. Dazu werden die Verbindungen der Formel I a und I b und/oder ihre physiologisch verträglichen Salze zusammen mit einem oder mehreren festen oder flüssigen galenischen Trägerstoffen und/oder Hilfsstoffen und, wenn gewünscht, in Kombination mit anderen Arzneimittelwirkstoffen in eine geeignete Darreichungsform bzw. Dosierungsform gebracht, die dann als Arzneimittel in der Humanmedizin oder Veterinärmedizin verwendet werden kann.

Arzneimittel, die erfindungsgemäße Verbindungen der Formel I a und I b und/oder ihre physiologisch verträglichen Salze enthalten, können oral, parenteral, z. B intravenös, rektal, durch Inhalation oder topisch appliziert werden, wobei die bevorzugte Applikation vom Einzelfall, z. B. dem jeweiligen Erscheinungsbild der zu behandelnden Erkrankung, abhängig ist.

Welche Hilfsstoffe für die gewünschte Arzneimittelformulierung geeignet sind, ist dem Fachmann auf Grund seines Fachwissens geläufig. Neben Lösemitteln, Gelbildnem, Suppositoriengrundlagen, Tablettenhilfsstoffen und anderen Wirkstoffträgem können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Die Verbindungen der Formel I a und I b können zur Erzielung einer vorteilhaften therapeutischen Wirkung auch mit anderen Arzneiwirkstoffen kombiniert werden. So sind in der Behandlung von Herz-Kreislauferkrankungen vorteilhafte Kombinationen mit herz-kreislaufaktiven Stoffen möglich. Als derartige, für Herz-Kreislauferkrankungen vorteilhafte Kombinationspartner kommen beispielsweise andere Antiarrhythmika, so Klasse I-, Klasse II- oder Klasse III- Antiarrhythmika, in Frage, wie beispielsweise IKₛ- oder IKᵣ-Kanalblocker, z.B. Dofetilid, oder weiterhin blutdrucksenkende Stoffe wie ACE- Inhibitoren (beispielsweise Enalapril, Captopril, Ramipril), Angiotensin-Antagonisten, K⁺-Kanalaktivatoren, sowie alpha- und beta-Rezeptorenblocker, aber auch sympathomimetische und adrenerg wirkende Verbindungen, sowie Na⁺/H⁺-Austausch-Inhibitoren, Calciumkanalantagonisten, Phosphodiesterasehemmer und andere positiv inotrop wirkende Stoffe, wie z. B. Digitalisglykoside, oder Diuretika.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran. Als Lösungsmittel für wässrige oder alkoholische Lösungen kommen z. B. Wasser, Ethanol oder Zuckerlösungen oder Gemische davon, in Betracht. Weitere Hilfsstoffe, auch für andere Applikationsformen, sind z. B. Polyethylenglykole und Polypropylenglykole.

Zur subkutanen oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Die Verbindungen der Formel I a und I b und ihre physiologisch verträglichen Salze können auch lyophilisiert werden und die erhaltenen Lyophilisate z. B. zur Herstellung von Injektions- oder Infusionspräparaten verwendet werden. Als Lösungsmittel kommen z. B. Wasser, physiologische Kochsalzlösung oder Alkohole, z. B. Ethanol, Propanol, Glycerin, in Betracht, daneben auch Zuckerlösungen wie Glucose- oder Mannitlösungen, oder auch Mischungen aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen der Wirkstoffe der Formel I a und I b oder ihrer physiologisch verträglichen Salze in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gewichtsprozent.

Die Dosierung des zu verabreichenden Wirkstoffs der Formel I a und I b bzw. der physiologisch verträglichen Salze davon hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder prophylaktisch therapiert wird. Üblicherweise beträgt die tägliche Dosis einer Verbindung der Formel I a und I b bei Verabreichung an einem etwa 75 kg schweren Patienten 0.001 mg/kg Körpergewicht bis 100 mg/kg Körpergewicht, bevorzugt 0.01 mg/kg Körpergewicht bis 20 mg/kg Körpergewicht. Die Dosis kann in Form einer Einzeldosis verabreicht werden oder in mehrere, z. B. zwei, drei oder vier Einzeldosen aufgeteilt werden. Insbesondere bei der Behandlung akuter Fälle von Herzrhythmusstörungen, beispielsweise auf einer Intensivstation, kann auch eine parenterale Verabreichung durch Injektion oder Infusion, z. B. durch eine intravenöse Dauerinfusion, vorteilhaft sein.

### Experimenteller Teil

### Liste der Abkürzungen

- Boc: tert.-Butyloxycarbonyl
- CDI: Carbonyldiimidazol
- DCC: Dicyclohexylcarbodiimid
- DMAP: 4-Dimethylaminopyridin
- DMF: N,N-Dimethylformamid
- DME: 1,2-Dimethoxyethan
- EDC: N-Ethyl-N'-(3-dimethylaminopropyl)-carbodiimidhydrochlorid
- Eq.: Moläquivalent
- HOBt: 1-Hydroxy-1H-benzotriazol
- Me: Methyl
- MeLi: Methyllithium (in Hexan)
- BuLi: Butyllithium (in Pentan)
- RT: Raumtemperatur
- RP-HPLC: Umkehrphasen-Hochleistungschromatographie
- THF: Tetrahydrofuran
- TFFH: Tetramethylfluoroamidiniumhexafluorophosphat

### Synthese der Boronsäuren der Formel II

Die Boronsäuren wurden gemäss Schema 3 synthetisiert - ihre Synthese wird anhand mehrerer Verbindungen demonstriert:

### 2-(tert-Butoxycarbonylamino-methyl)-phenylboronsäure (Verbindung 1)

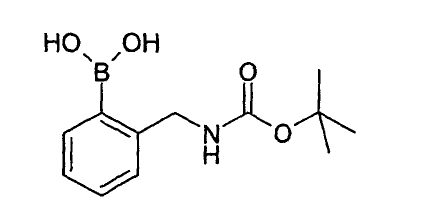

N-Boc-2-Brombenzylamin (5.72 g, 20 mmol) wurde in THF unter Argon gelöst , auf - 78°C gekühlt, mit 13.75 ml MeLi (1.6 M in Hexan, 22 mmol) versetzt, nach 1 h mit 28 ml (1.5 M in Pentan, 42 mmol) tert. BuLi versetzt und nach einer weiteren Stunde bei - 78°C erfolgte Zugabe von Borsäuretrimethylester (9.0 ml, 80 mmol). Nach Erwärmen auf Raumtemperatur wurde mit verdünnter Salzsäure bis pH 6 versetzt, mit Dichlormethan extrahiert, die organische Phase mit gesättigter NaCl-Lösung gewaschen und getrocknet. Man erhielt 5.1 g (100%) eines hellgelben festen Schaumes. MS (FAB, Probe mit Glycerin versetzt): m/z = 308 (M + 57), 252 (M +1).

### (R)-2-(1-tert-Butoxycarbonylaminoethyl)-phenylboronsäure (Verbindung 2)

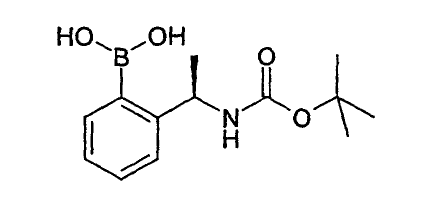

2.2 g (10 mmol) N-Boc-(R)-Phenethylamin wurden in 50 ml wasserfreiem THF gelöst, auf -78°C gekühlt und tropfenweise mit 14 ml (1.5 M Lösung in Pentan, 21 mmol) tert. Butyllithium versetzt. Während 2 h wurde auf -20°C erwärmt, anschließend 4.5 ml (40 mmol) Borsäuretrimethylester zugegeben und auf Raumtemperatur erwärmt. Die Lösung wurde auf 0°C gekühlt, mit 10% HCl bis auf pH 6 angesäuert, die wässrige Phase mit Dichlormethan extrahiert, die vereinigten organischen Phasen mit gesättigter NaCl-Lösung gewaschen, getrocknet und eingeengt. Man erhielt 2.0 g (75%) eines hellgelben festen Schaumes der ohne weitere Reinigung verwendet wurde. MS (FAB, Probe mit Glycerin versetzt): m/z = 322 (M + 57), 266 (M +1).

### Synthese aromatischer Halogenide der Formeln III a, b

Allgemeine Arbeitsvorschrift für die Synthese der Verbindungen der Formel VII a, b mit Thionylchlorid:
2.5 mmol Säure der Formel X werden mit 3 ml Thionylchlorid 4 h zum Rückfluss erhitzt und anschliessend eingeengt. Das rohe Reaktionsprodukt wird zweimal mit Toluol koevaporiert, in 12.5 ml Dichlormethan aufgenommen und mit 3 mmol des Amins NHR(3)R(4) und 5.5 mmol Triethylamin versetzt. Die Mischung wird über Nacht gerührt, mit NaHCO₃-Lösung gewaschen, getrocknet und eingeengt. Man erhält 1.5 bis 2.5 mmol des gewünschten Amids III das ohne weitere Reinigung eingesetzt werden kann.

### Amide III a, b gemäß allgemeiner Arbeitsvorschrift

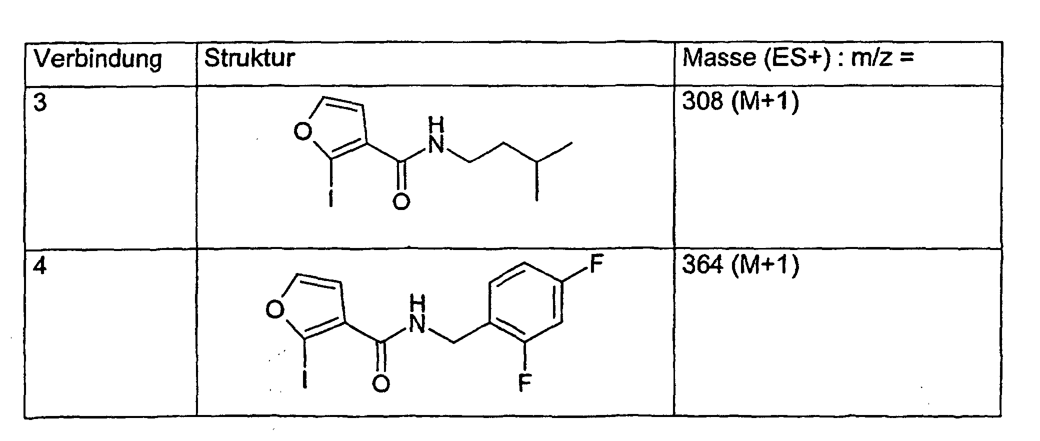

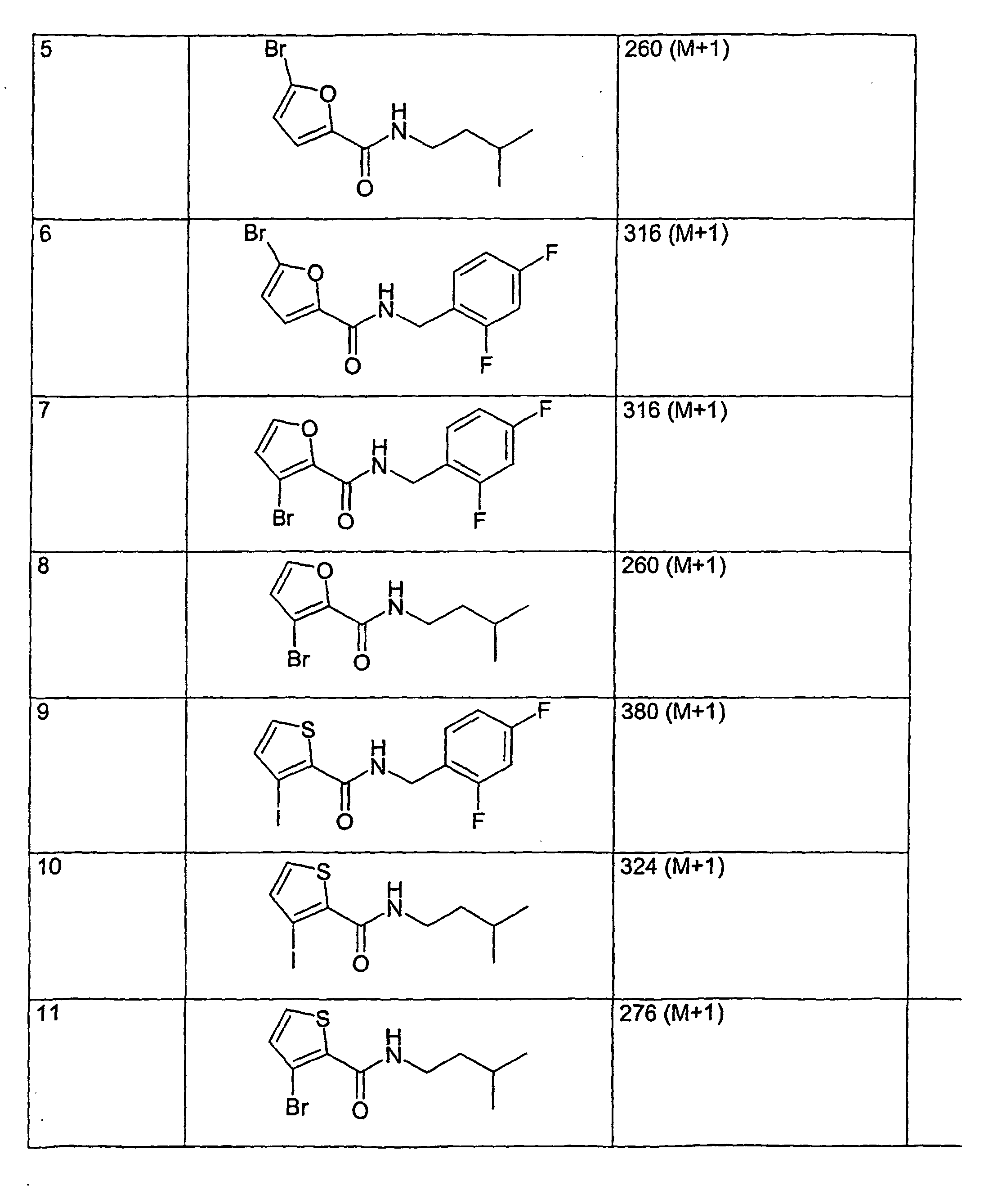

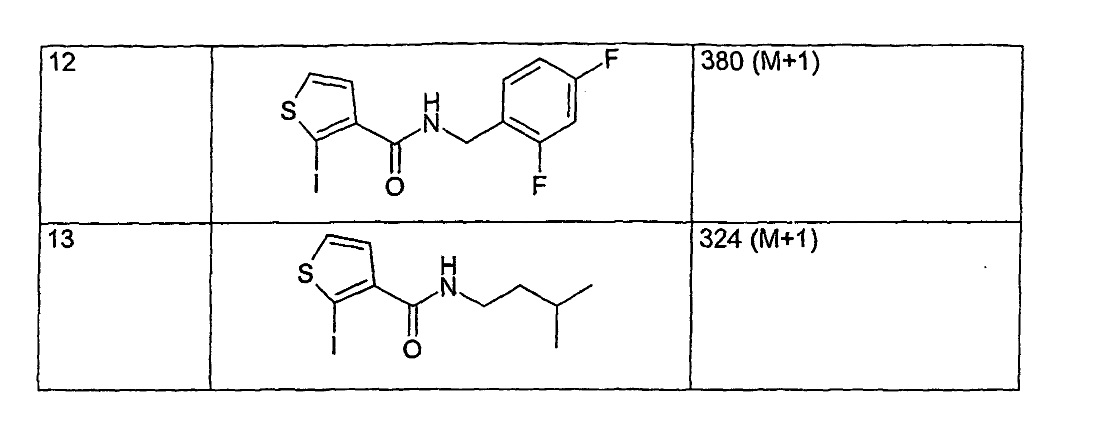

Aufbau der arylierten Furan- und Thiophencarbonsäureamide durch Palladium-katalysierte Suzuki-Kupplung zu den Verbindungen der Formeln IV a, b

### Allgemeine Arbeitsvorschrift:

Zu mit Argon begastem 1,2-Dimethoxyethan (10 ml/ mmol Bromid III a, b) wurden 0. 05 eq. Tetrakis-triphenylphosphin-Palladium und 1 eq. des entsprechenden Bromids III a, b zugegeben. Nach 10 min wurde 1.5 eq. der entsprechenden Boronsäure II zugesetzt und zuletzt 2 eq. einer 2 molaren Natriumcarbonatlösung. Es wurde 18h zum Rückfluss unter Argon erhitzt, abgekühlt und mit Methylenchlorid verdünnt. Die Mischung wurde mit Wasser und gesättigter Kochsalzlösung gewaschen, über Natriumsulfat getrocknet, eingeengt und chromatographisch gereinigt. Bei der Reinigung per RP-HPLC wurden basische Verbindungen als Trifluoracetate isoliert.

### Beispiel für ein aryliertes Thiophencarbonsäureamid der Formel IV a:

### Beispiel 1: {2-[2-(3-Methylbutylcarbamoyl)-thiophen-3-yl]-benzyl}-carbaminsäure-tert-butylester

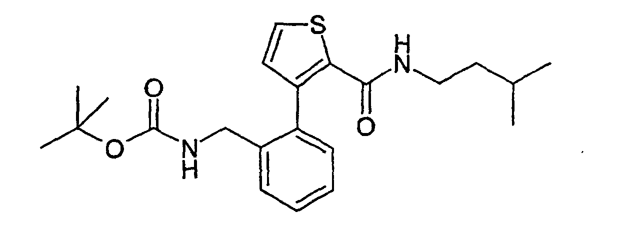

10 ml 1,2-Dimethoxyethan wurden mit Argon begast, 58 mg (0.05 mmol) Pd(PPh₃)₄ und 276 mg (1 mmol) 3-Bromthiophen-2-carbonsäure-3-methylbutylamid zugegeben. Nach 10 min wurden 377 mg (1.5 mmol) 2-(tert-Butoxycarbonylamino-methyl)-phenylboronsäure und zuletzt 1 ml einer 2M Natriumcarbonatlösung zugegeben. Die Mischung wurde unter Argon 18 h zum Rückfluss erhitzt, nach dem Abkühlen mit Dichlormethan verdünnt und mit Wasser gewaschen. Die organische Phase (schwarzes, zähes Öl) wurde getrocknet, eingeengt und chromatographisch per RP-HPLC gereinigt. Man erhielt 51 mg (13 %) eines zähen farblosen Öls. MS (ES+): m/z = 403 (M + 1), 303 (M- 99). ¹H-NMR (CDCl₃): δ = 7.55 - 7.21 (5H, m), 6.90 (1 H, d, J = 4.8 Hz), 5.58 (1 H, br s), 4.82 (1 H, br s), 4.10 (2H, d, J = 6.2 Hz), 3.18 (2H, m), 1.40 (9H, s), 1.16 (1 H, m), 1.07 (2H, q, J = 7.0 Hz), 0.75 (6H, d, J = 6.2 Hz).

### Weitere Beispiele für arylierte Furan- und Thiophencarbonsäureamide der Formel IV a und IV b (nach Methode A)

Entsprechend der oben angeführten allgemeinen Arbeitsvorschrift wurden folgende Beispiele synthetisiert:

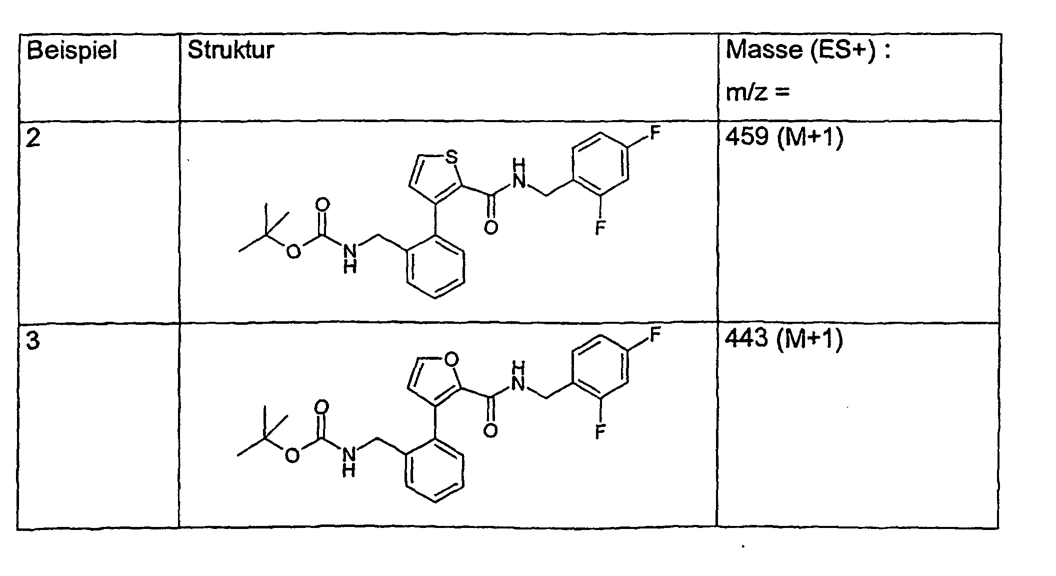

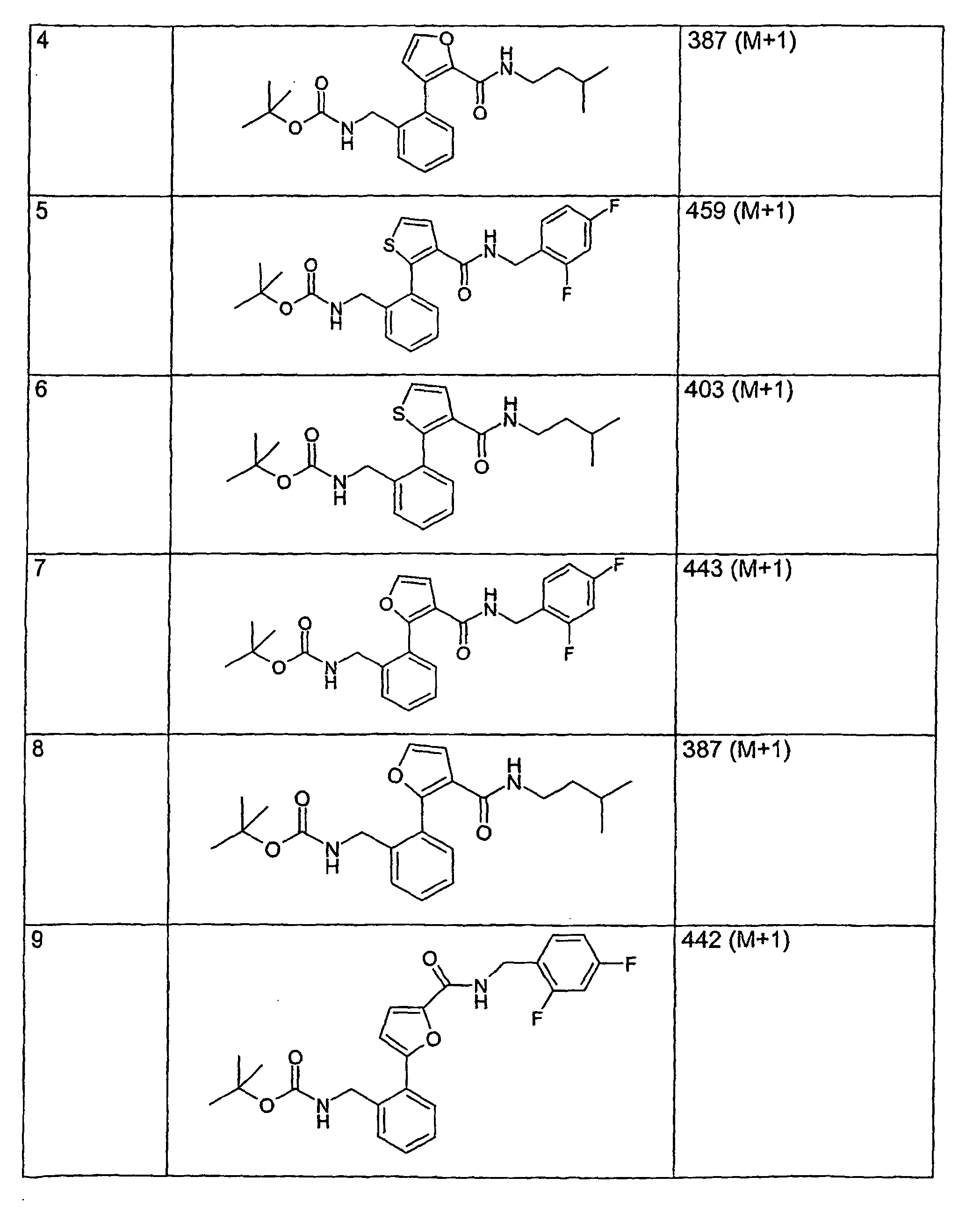

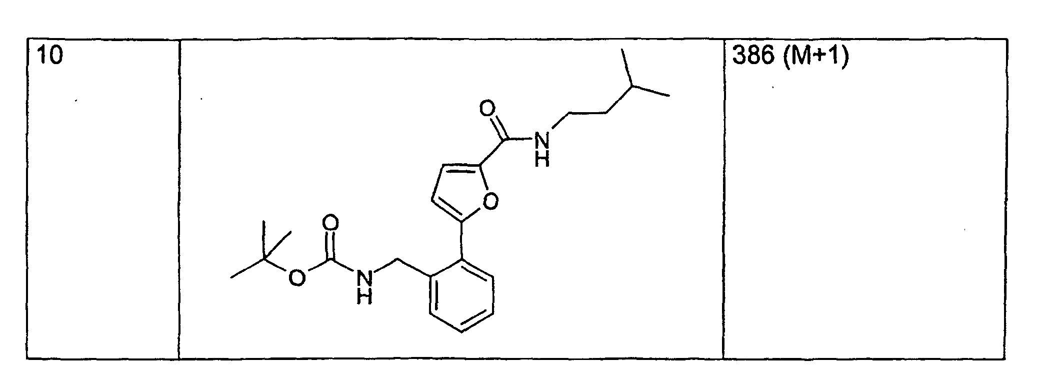

### Abspaltung der Boc-Schutzgruppe zu den Aminen V (Schema 1 und 2)

### Allgemeine Arbeitsvorschrift

1 eq. der N-Boc-Verbindung wird in Dichlormethan/Trifluoressigsäure (3/1, 10 ml/mmol) gelöst und bei Raumtemperatur 3 h gerührt. Anschliessend wird am Rotationsverdampfer eingeengt und mit Toluol koevaporiert. Die Amine V werden ohne weitere Reinigung für weitere Umsetzungen verwendet. Alle Verbindungen wurden durch Massenspektrometrie charakterisiert.

### Umsetzung der Amine V mit verschiedenen Reagenzien zu den Zielverbindungen I

Allgemeine Arbeitsvorschrift für die Umsetzung zu Carbamaten der Formel I 1 eq. des Amins V wird in Dichlormethan (ca. 10 ml/mmol) gelöst und mit 1.2 eq (2.2 eq. bei Verwendung des Trifluoracetates) Triethylamin und 1.2 eq. des Succinimidylcarbonats (oder wahlweise des entsprechenden Chloroformiats) versetzt und über Nacht gerührt. Es wird mit Dichlormethan verdünnt und mit NaHCO₃-Lösung gewaschen. Die organische Phase wird getrocknet, eingeengt und, falls erforderlich, per RP-HPLC gereinigt.

### Beispiel 11: {2-[2-(3-Methyl-butylcarbamoyl)-thiophen-3-yl]-benzyl}carbaminsäurebenzylester

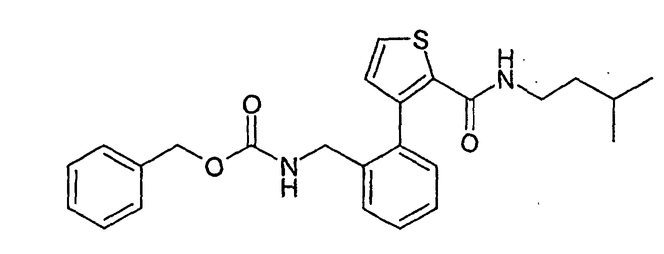

27 mg (0.09 mmol) 3-(2-Aminomethylphenyl)-thiophen-2-carbonsäure (3-methyl-butyl)-amid wurden in 3 ml trockenem Dichlormethan gelöst, mit 10 mg (0.1 mmol) Triethylamin und 24 mg (0.1 mmol) Benzyloxycarbonyloxysuccinimid versetzt. Nach 18 h Reaktionszeit wurde mit 20 ml Dichlormethan verdünnt, mit gesättigter NaHCO₃-Lösung gewaschen und die organische Phase getrocknet und eingeengt. Nach der Reinigung per RP-HPLC erhielt man 27 mg (70%) einer farblosen Substanz. MS (ES+): m/z = 437 (M + 1). ¹H-NMR (CDCl₃): δ = 7.54 - 7.24 (10H, m), 6.90 (1 H, d, J = 4.8 Hz), 5.52 (1 H, br s), 5.05 (3H, br s), 4.17 (2H, d, J = 6.2 Hz), 3.16 (2H, m), 1.14 (1 H, m), 1.07 (2H, q, J = 7.0 Hz), 0.73 (6H, d, J = 6.2 Hz).

Weitere Beispiele die entsprechend der Arbeitsvorschrift daraestellt wurden:

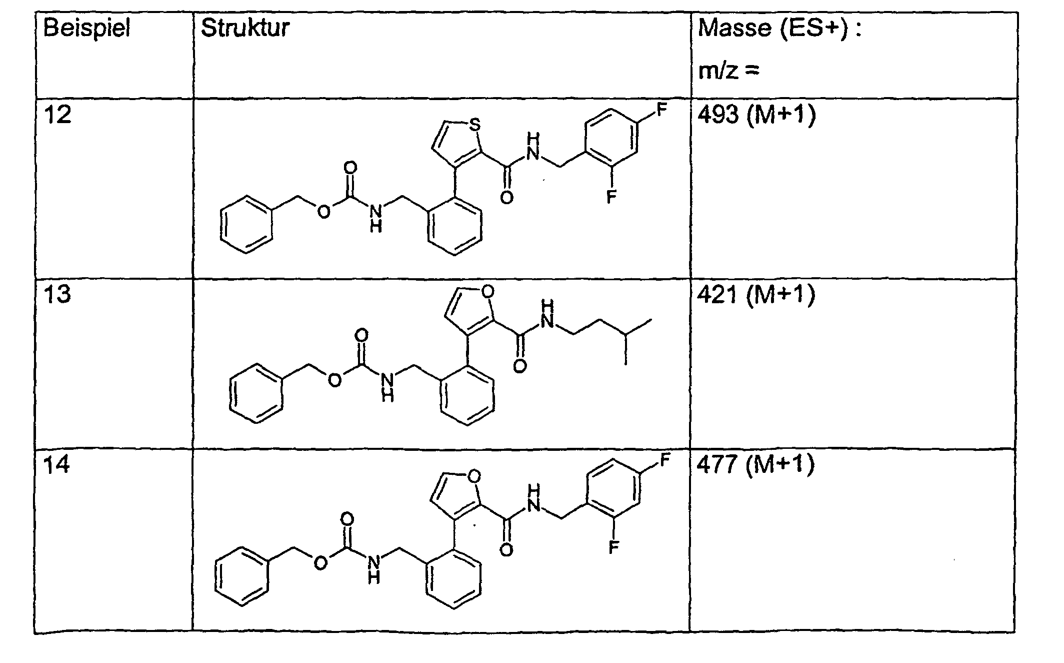

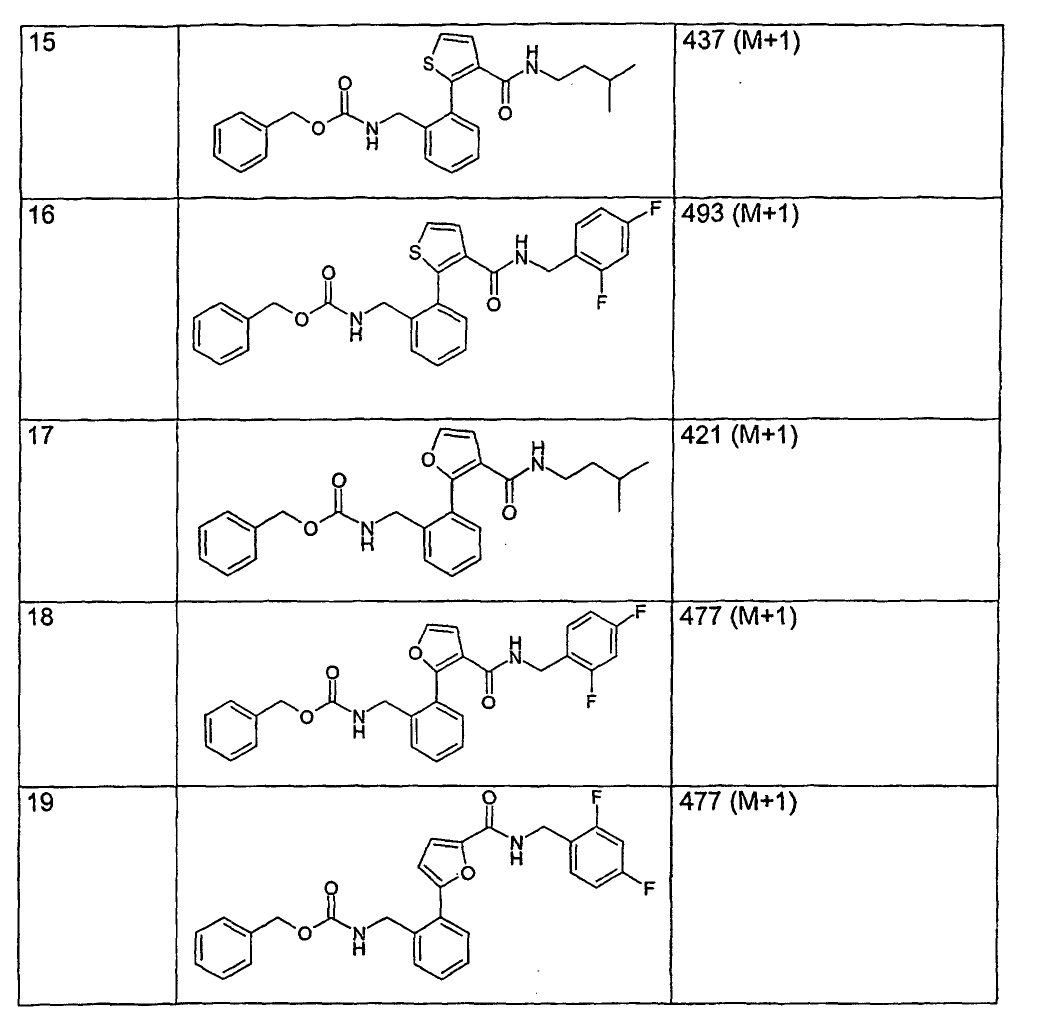

Allgemeine Arbeitsvorschrift für die Umsetzung zu Amiden der Formel I
A) 1 eq. des Amins V wird in Dichlormethan (ca. 10 ml/mmol) gelöst, mit 1.2 eq (2.2 eq. bei Verwendung des Trifluoracetates) Diisopropylethylamin und 1.2 eq. des Säurechlorids versetzt und über Nacht gerührt. Es wird mit Dichlormethan verdünnt und mit NaHCO₃-Lösung gewaschen. Die organische Phase wird getrocknet, eingeengt und, falls erforderlich, per RP-HPLC gereinigt.
B) 1 eq. des Amins V wird in Dichlormethan (ca. 10 ml/mmol) gelöst, mit 1.2 eq (2.2 eq. bei Verwendung des Trifluoracetates) Diisopropylethylamin, mit 1.2 eq. der Säure und 1.2 eq. TFFH versetzt und über Nacht gerührt. Es wird mit Dichlormethan verdünnt und mit NaHCO₃-Lösung gewaschen. Die organische Phase wird getrocknet, eingeengt und, falls erforderlich, per RP-HPLC gereinigt.

### Beispiele gemäss der allgemeinen Arbeitsvorschrift A oder B:

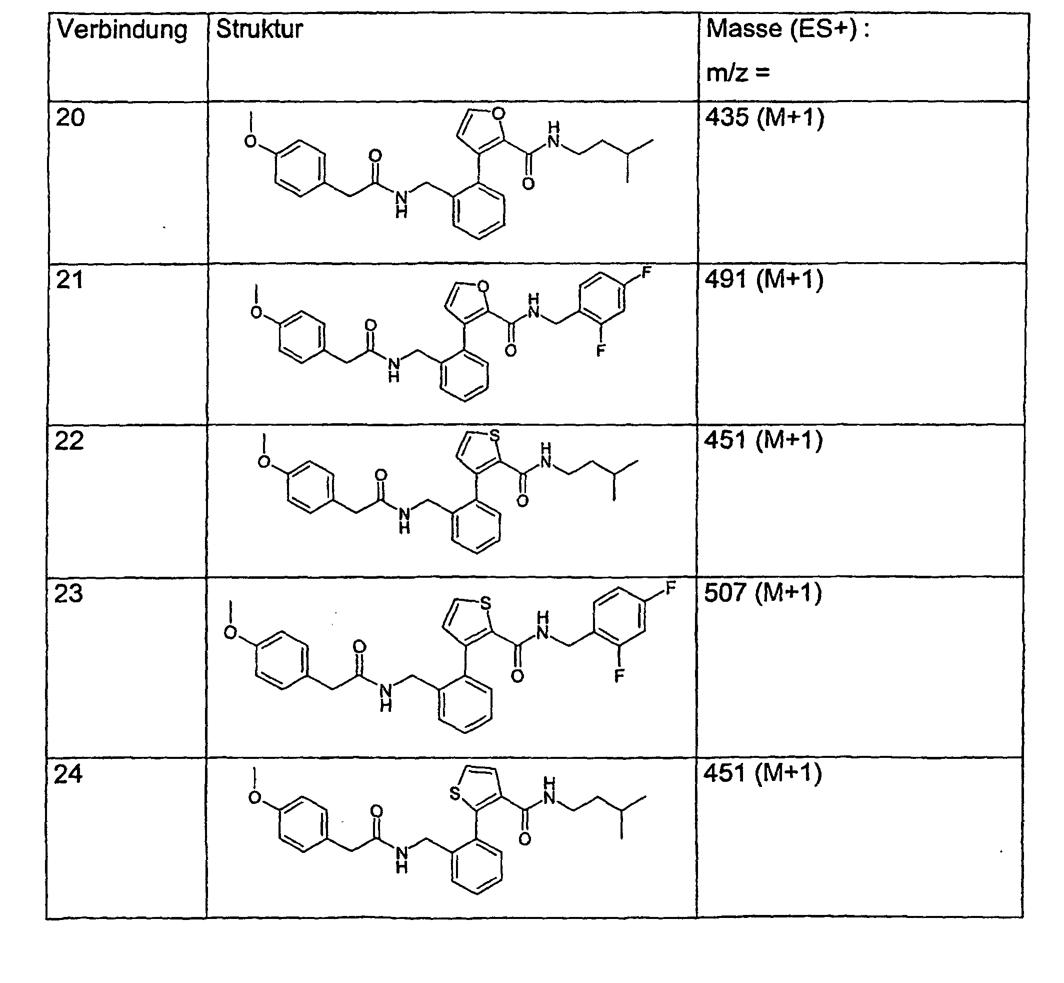

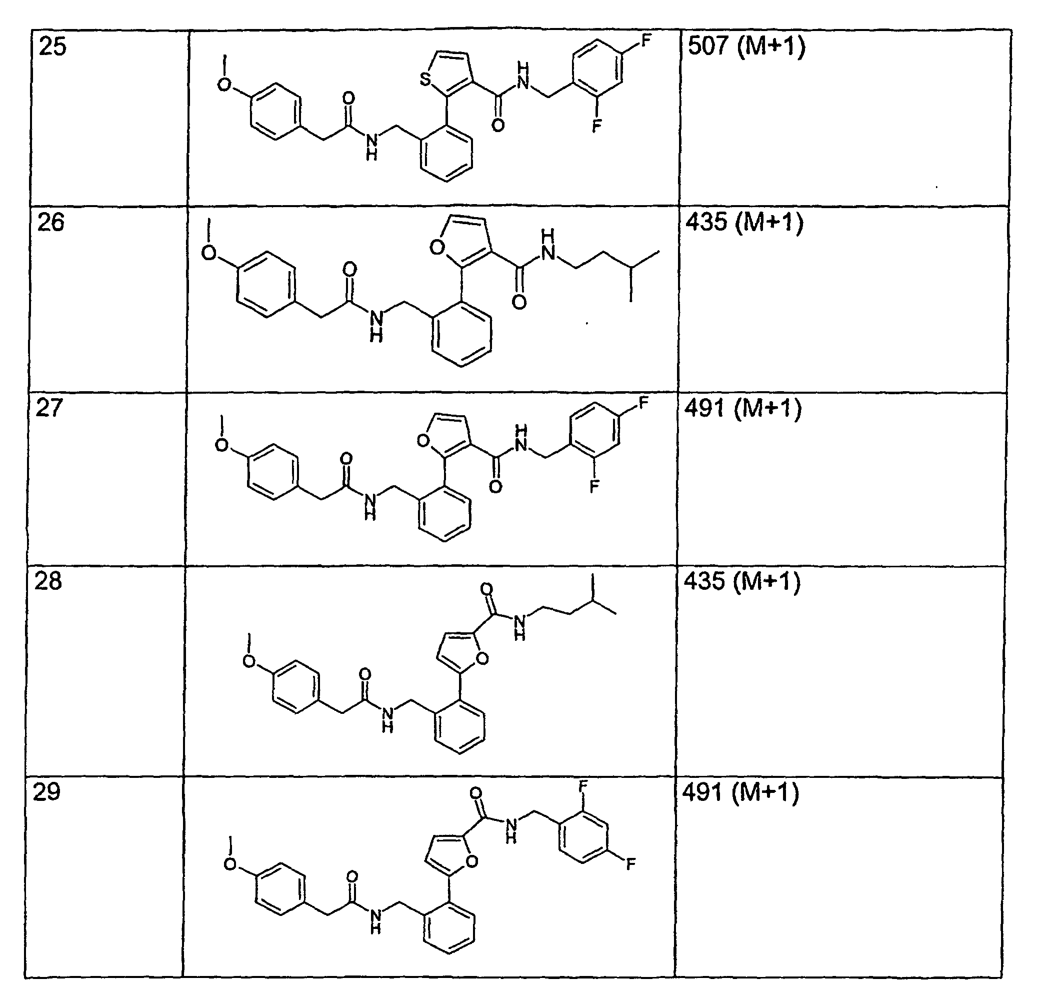

### Pharmakologische Untersuchungen

Kv1.5-Kanäle aus dem Menschen wurden in Xenopus Oozyten expremiert. Hierfür wurden zuerst Oozyten aus Xenopus laevis isoliert und defollikuliert. Anschließend wurde in diese Oozyten in vitro synthetisierte Kv1.5 codierende RNA injiziert. Nach 1 - 7 Tagen Kv1.5-Proteinexpression wurden an den Oozyten mit der Zwei-Mikroelektroden Voltage-Clamp Technik Kv1.5-Ströme gemessen. Die Kv1.5-Kanäle wurden hierbei in der Regel mit 500 ms dauernden Spannungssprüngen auf 0 mV und 40 mV aktiviert. Das Bad wurde mit einer Lösung der nachfolgenden Zusammensetzung durchspült: NaCl 96 mM, KCl 2 mM, CaCl₂ 1,8 mM, MgCl₂ 1 mM, HEPES 5 mM (titriert mit NaOH auf pH 7,4). Diese Experimente wurden bei Raumtemperatur durchgeführt. Zur Datenerhebung und Analyse wurden eingesetzt: Geneclamp Verstärker (Axon Instruments, Foster City, USA) und MacLab D/A-Umwandler und Software (ADInstruments, Castle Hill, Australia). Die erfindungsgemäßen Substanzen wurden getestet, indem sie in unterschiedlichen Konzentrationen der Badlösung zugefügt wurden. Die Effekte der Substanzen wurden als prozentuale Inhibition des Kv1.5-Kontrollstromes berechnet, der erhalten wurde, wenn der Lösung keine Substanz zugesetzt wurde. Die Daten wurden anschließend mit der Hill-Gleichung extrapoliert, um die Hemmkonzentrationen IC₅₀ für die jeweiligen Substanzen zu bestimmen.

Auf diese Weise wurden für die nachfolgend aufgeführten Verbindungen folgende IC₅₀-Werte bestimmt

| Bsp. | IC₅₀ [µM] | Bsp. | IC₅₀ [µM] | Bsp. | IC₅₀ [µM] | Bsp. | IC₅₀ [µM] |
|---|---|---|---|---|---|---|---|
| 1 | 4 | 2 | 5 | 3 | 9 | 4 | 10 |
| 5 | 3.1 | 6 | <100 | 7 | 10 | 8 | 5.2 |
| 9 | <100 | 10 | <100 | 11 | 3 | 12 | 2 |
| 13 | 5.6 | 14 | <100 | 15 | 1.2 | 16 | 1.9 |
| 17 | 2.2 | 18 | 5.2 | 19 | <100 | 20 | <100 |
| 21 | 3.9 | 22 | 1.7 | 23 | 1.2 | 24 | 2.2 |
| 25 | 1.5 | 26 | 3.9 | 27 | 6.6 | 28 | <100 |
| 29 | 4.2 | | | | | | |

## Patentansprüche

1. Verbindungen der Formel I a und I b, 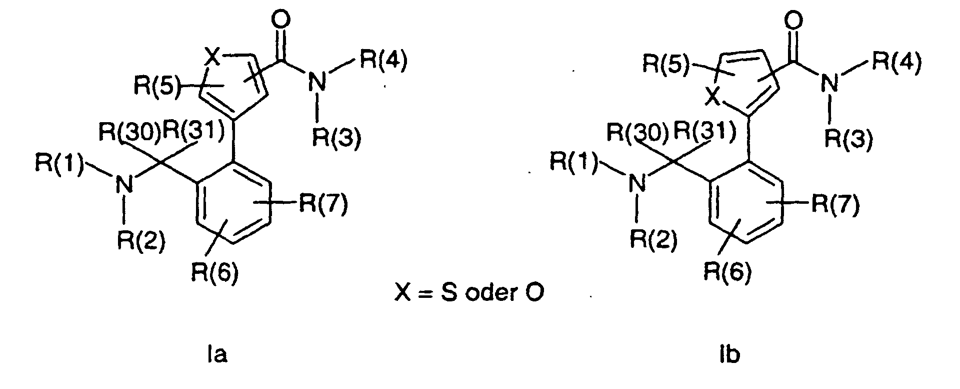 worin bedeuten:
X Sauerstoff oder Schwefel;
R(1) C(O)OR(9), SO₂R(10), COR(11), C(O)NR(12)R(13) oder C(S)NR(12)R(13);
R(9), R(10), R(11) und R(12) unabhängig voneinander CₓH₂ₓ-R(14);
x 0, 1, 2, 3 oder 4,
wobei x nicht 0 sein kann, wenn R(14) OR(15) oder SO₂Me bedeutet;
R(14) Alkyl mit 1, 2, 3, 4, 5 oder 6 C-Atomen, Cycloalkyl mit 3, 4, 5, 6, 7, 8, 9, 10 oder 11 C-Atomen, CF₃, C₂F₅, C₃F₇, CH₂F, CHF₂, OR(15), SO₂Me, Phenyl, Naphthyl, Biphenylyl, Furyl, Thienyl oder ein N-haltiger Heteroaromat mit 1, 2, 3, 4, 5, 6, 7, 8 oder 9 C-Atomen,
wobei Phenyl, Naphthyl, Biphenylyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(15) Alkyl mit 1, 2, 3, 4 oder 5 C-Atomen, Cycloalkyl mit 3, 4, 5 oder 6 C-Atomen, CF₃ oder Phenyl, das unsubstituiert ist oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(13) Wasserstoff, Alkyl mit 1, 2, 3 oder 4 C-Atomen oder CF₃;
R(2) Wasserstoff, Alkyl mit 1, 2, 3 oder 4 C-Atomen oder CF₃;
R(3) C_{y}H_{2y}-R(16);
y 0, 1, 2, 3 oder 4,
wobei y nicht 0 sein kann, wenn R(16) OR(17) oder SO₂Me bedeutet;
R(16) Alkyl mit 1, 2, 3, 4, 5 oder 6 C-Atomen, Cycloalkyl mit 3, 4, 5, 6, 7, 8, 9, 10 oder 11 C-Atomen, CF₃, C₂F₅, C₃F₇, CH₂F, CHF₂, OR(17), SO₂Me, Phenyl, Naphthyl, Furyl, Thienyl oder ein N-haltiger Heteroaromat mit 1, 2, 3, 4, 5, 6, 7, 8 oder 9 C-Atomen,
wobei Phenyl, Naphthyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino.
R(17) Wasserstoff, Alkyl mit 1, 2, 3, 4 oder 5 C-Atomen, Cycloalkyl mit 3, 4, 5 oder 6 C-Atomen, CF₃, Phenyl oder 2-, 3- oder 4-Pyridyl,
wobei Phenyl oder 2-, 3- oder 4- Pyridyl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
oder
R(3) CHR(18)R(19);
R(18) Wasserstoff oder C_{z}H_{2z}-R(16), wobei R(16) wie oben angegeben definiert ist;
z 0, 1, 2 oder 3;
R(19) COOH, CONH₂, CONR(20)R(21), COOR(22) oder CH₂OH;
R(20) Wasserstoff, Alkyl mit 1, 2, 3, 4 oder 5 C-Atomen, CᵥH₂ᵥ-CF₃ oder C_{w}H_{2w}-Phenyl,
wobei der Phenylring unsubstituiert ist oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
v 0, 1, 2 oder 3;
w 0, 1, 2 oder 3;
R(21) Wasserstoff oder Alkyl mit 1, 2, 3, 4 oder 5 C-Atomen;
R(22) Alkyl mit 1, 2, 3, 4 oder 5 C-Atomen;
R(4) Wasserstoff, Alkyl mit 1, 2, 3, 4, 5 oder 6 C-Atomen oder CF₃;
oder
R(3) und R(4) gemeinsam eine Kette von 4 oder 5 Methylengruppen, von denen eine Methylengruppe durch -O-, -S-, -NH-, -N(Methyl)- oder -N(Benzyl)- ersetzt sein kann;
R(5), R(6) und R(7) unabhängig voneinander Wasserstoff, F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl oder Methylsulfonylamino.
R(30) und R(31) unabhängig voneinander Wasserstoff oder Alkyl mit 1, 2 oder 3 C-Atomen;
oder
R(30) und R(31) gemeinsam Sauerstoff oder eine Kette von 2 Methylengruppen;
sowie ihre pharmazeutisch akzeptablen Salze.

2. Verbindungen der Formel I a und I b nach Anspruch 1, in der bedeuten:
X Sauerstoff oder Schwefel;
R(1) C(O)OR(9), SO₂R(10), COR(11) oder C(O)NR(12)R(13);
R(9), R(10), R(11) und R(12) unabhängig voneinander CₓH₂ₓ-R(14);
x 0, 1, 2, 3 oder 4,
wobei x nicht 0 sein kann, wenn R(14) OR(15) bedeutet
R(14) Alkyl mit 1, 2, 3, 4, C-Atomen, Cycloalkyl mit 3, 4, 5, 6, 7, 8, 9 C-Atomen, CF₃, OR(15), Phenyl, Naphthyl, Biphenylyl, Furyl, Thienyl oder ein N-haltiger Heteroaromat mit 1, 2, 3, 4, 5, 6, 7, 8 oder 9 C-Atomen,
wobei Phenyl, Naphthyl, Biphenylyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(15) Alkyl mit 1, 2, 3, 4 oder 5 C-Atomen, Cycloalkyl mit 3, 4, 5 oder 6 C-Atomen, CF₃ oder Phenyl, das unsubstituiert ist oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(13) Wasserstoff, Alkyl mit 1, 2, 3 oder 4 C-Atomen oder CF₃;
R(2) Wasserstoff, Alkyl mit 1, 2, 3 oder 4 C-Atomen oder CF₃;
R(3) C_{y}H_{2y}-R(16);
y 0, 1, 2, 3 oder 4,
wobei y nicht 0 sein kann, wenn R(16) OR(17) oder SO₂Me bedeutet;
R(16) Alkyl mit 1, 2, 3, 4, 5 oder 6 C-Atomen, Cycloalkyl mit 3, 4, 5, 6, 7, 8, 9, C-Atomen, CF₃, OR(17), SO₂Me, Phenyl, Naphthyl, Furyl, Thienyl oder ein N-haltiger Heteroaromat mit 1, 2, 3, 4, 5, 6, 7, 8 oder 9 C-Atomen,
wobei Phenyl, Naphthyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(17) Wasserstoff, Alkyl mit 1, 2, 3, 4 oder 5 C-Atomen, Cycloalkyl mit 3, 4, 5 oder 6 C-Atomen, CF₃, Phenyl oder 2-, 3- oder 4-Pyridyl,
wobei Phenyl oder 2-, 3- oder 4- Pyridyl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
oder
R(3) CHR(18)R(19);
R(18) Wasserstoff oder C_{z}H_{2z}-R(16), wobei R(16) wie oben angegeben definiert ist;
z 0, 1, 2 oder 3;
R(19) CONH₂, CONR(20)R(21), COOR(22) oder CH₂OH;
R(20) Wasserstoff, Alkyl mit 1, 2, 3, 4 oder 5 C-Atomen, CᵥH₂ᵥ-CF₃ oder C_{w}H_{2w}-Phenyl,
wobei der Phenylring unsubstituiert ist oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
v 0, 1, 2 oder 3;
w 0, 1, 2 oder 3;
R(21) Wasserstoff oder Alkyl mit 1, 2, 3, 4 oder 5 C-Atomen;
R(22) Alkyl mit 1, 2, 3, 4 oder 5 C-Atomen;
R(4) Wasserstoff, Alkyl mit 1, 2, 3, 4, 5 oder 6 C-Atomen oder CF₃;
R(5), R(6) und R(7) unabhängig voneinander Wasserstoff, F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl oder Methylsulfonylamino;
R(30) und R(31) unabhängig voneinander Wasserstoff oder Alkyl mit 1, 2 oder 3 C-Atomen;
oder
R(30) und R(31) eine Kette von 2 Methylengruppen
sowie ihre pharmazeutisch akzeptablen Salze.

3. Verbindungen der Formel I a und I b nach Ansprüchen 1 und 2 in der bedeuten:
X Sauerstoff oder Schwefel;
R(1) C(O)OR(9), SO₂R(10), COR(11) oder C(O)NR(12)R(13);
R(9), R(10), R(11) und R(12) unabhängig voneinander CₓH₂ₓ-R(14);
x 0, 1, 2, 3 oder 4,
wobei x nicht 0 sein kann, wenn R(14) OR(15) bedeutet;
R(14) Alkyl mit 1, 2, 3, 4, 5 oder 6 C-Atomen, Cycloalkyl mit 3, 4, 5, 6, 7, 8, 9, 10 oder 11 C-Atomen, CF₃, C₂F₅, C₃F₇, CH₂F, CHF₂, OR(15), Phenyl, Naphthyl, Biphenylyl, Furyl, Thienyl oder ein N-haltiger Heteroaromat mit 1, 2, 3, 4, 5, 6, 7, 8 oder 9 C-Atomen,
wobei Phenyl, Naphthyl, Biphenylyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(15) Alkyl mit 1, 2, 3, 4 oder 5 C-Atomen, Cycloalkyl mit 3, 4, 5 oder 6 C-Atomen, CF₃ oder Phenyl, das unsubstituiert ist oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(13) Wasserstoff;
R(2) Wasserstoff oder Alkyl mit 1, 2 oder 3 C-Atomen;
R(3) CHR(18)R(19);
R(18) Wasserstoff oder C₂H_{2z}-R(16),
R(16) Alkyl mit 1, 2, 3, 4, 5 oder 6 C-Atomen, Cycloalkyl mit 3, 4, 5, 6, 7, 8, 9, C-Atomen, CF₃, OR(17), SO₂Me, Phenyl, Naphthyl, Furyl, Thienyl oder ein N-haltiger Heteroaromat mit 1, 2, 3, 4, 5, 6, 7, 8 oder 9 C-Atomen,
wobei Phenyl, Naphthyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(17) Wasserstoff, Alkyl mit 1, 2, 3, 4 oder 5 C-Atomen, Cycloalkyl mit 3, 4, 5 oder 6 C-Atomen, CF₃, Phenyl oder 2-, 3- oder 4-Pyridyl,
wobei Phenyl oder 2-, 3- oder 4- Pyridyl unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe. NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
z 0, 1, 2 oder 3;
R(19) CONH₂, CONR(20)R(21), COOR(22) oder CH₂OH;
R(20) Wasserstoff, Alkyl mit 1, 2, 3, 4 oder 5 C-Atomen, CᵥH₂ᵥ-CF₃ oder C_{w}H_{2w}-Phenyl,
wobei der Phenylring unsubstituiert ist oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
v 0, 1, 2 oder 3;
w 0, 1, 2 oder 3;
R(21) Wasserstoff oder Alkyl mit 1, 2, 3, 4 oder 5 C-Atomen;
R(22) Alkyl mit 1, 2, 3, 4 oder 5 C-Atomen;
R(4) Wasserstoff, Alkyl mit 1 oder 2 C-Atomen
R(5), R(6) und R(7) unabhängig voneinander Wasserstoff, F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl oder Methylsulfonylamino;
R(30) und R(31) unabhängig voneinander Wasserstoff oder Methyl;
sowie ihre pharmazeutisch akzeptablen Salze.

4. Verbindungen der Formel I a und I b nach Ansprüchen 1 - 3, in der bedeuten:
X Sauerstoff oder Schwefel;
R(1) C(O)OR(9), SO₂R(10), COR(11) oder C(O)NR(12)R(13);
R(9), R(10), R(11) und R(12) unabhängig voneinander CₓH₂ₓ-R(14);
x 0, 1, 2, 3 oder 4,
R(14) Alkyl mit 1, 2, 3, 4, 5 oder 6 C-Atomen, Cycloalkyl mit 3, 4, 5, 6, 7, 8, 9, 10 oder 11 C-Atomen, CF₃, C₂F₅, C₃F₇, CH₂F, CHF₂, SO₂Me, Phenyl, Naphthyl, Biphenylyl, Furyl, Thienyl oder ein N-haltiger Heteroaromat mit 1, 2, 3, 4, 5, 6, 7, 8 oder 9 C-Atomen,
wobei Phenyl, Naphthyl, Biphenylyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, OCF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(13) Wasserstoff;
R(2) Wasserstoff oder Methyl;
R(3) C_{y}H_{2y}-R(16);
y 0, 1, 2, 3 oder 4;
wobei y nicht 0 sein kann, wenn R(16) OR(17) bedeutet;
R(16) Alkyl mit 1, 2, 3, 4, 5 oder 6 C-Atomen, Cycloalkyl mit 3, 4, 5, 6, 7, 8, 9, 10 oder 11 C-Atomen, CF₃, C₂F₅, C₃F₇, CH₂F, CHF₂, OR(17), SO₂Me, Phenyl, Naphthyl, Furyl, Thienyl oder ein N-haltiger Heteroaromat mit 1, 2, 3, 4, 5, 6, 7, 8 oder 9 C-Atomen,
wobei Phenyl, Naphthyl, Furyl, Thienyl und der N-haltige Heteroaromat unsubstituiert sind oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(17) Wasserstoff, Alkyl mit 1, 2, 3, 4 oder 5 C-Atomen, Cycloalkyl mit 3, 4, 5 oder 6 C-Atomen, CF₃, Phenyl oder 2-, 3- oder 4-Pyridyl,
wobei Phenyl oder 2-, 3- oder 4- Pyridyl unsubstituiert sind oder substituiert mit 1, 2 oder 3 Substituenten ausgewählt aus der Gruppe bestehend aus F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sulfamoyl, Methylsulfonyl und Methylsulfonylamino;
R(4) Wasserstoff oder Alkyl mit 1 oder 2 C-Atomen;
R(5), R(6) und R(7) unabhängig voneinander Wasserstoff, F, Cl, Br, I, CF₃, NO₂, CN, COOMe, CONH₂, COMe, NH₂, OH, Alkyl mit 1, 2, 3 oder 4 C-Atomen, Alkoxy mit 1, 2, 3 oder 4 C-Atomen, Dimethylamino, Sutfamoyl, Methylsulfonyl oder Methylsulfonylamino;
R(30) und R(31) unabhängig voneinander Wasserstoff oder Methyl;
sowie ihre pharmazeutisch akzeptablen Salze.

5. Verbindungen der Formel I a oder I b nach Ansprüchen 1 - 4, in der bedeuten:
R(1) C(O)OR(9), SO₂R(10), COR(11) oder C(O)NR(12)R(13);
R(9), R(10), R(11) und R(12) unabhängig voneinander CₓH₂ₓ-R(14);
x 0, 1, 2 oder 3;
R(14) Alkyl mit 1, 2, 3 oder 4 C-Atomen, Cycloalkyl mit 3, 4, 5, 6, 7, 8 oder 9 c-Atomen, CF₃, Phenyl oder Pyridyl,
wobei Phenyl und Pyridyl unsubstituiert sind oder substituiert mit 1 oder 2 Substituenten ausgewählt aus der Gruppe bestehend aus F, Cl, Br, I, CF₃, OCF₃, OH, Alkyl mit 1, 2, 3, Alkoxy mit 1 oder 2 C-Atomen;
R(13) Wasserstoff:
R(2) Wasserstoff;
R(3) C_{y}H_{2y}-R(16);
y 0, 1 oder 2;
R(16) Alkyl mit 1, 2 oder 3 C-Atomen, Cycloalkyl mit 5 oder 6 C-Atomen, CF₃, Phenyl oder Pyridyl,
wobei Phenyl und Pyridyl unsubstituiert sind oder substituiert mit 1 oder 2 Substituenten ausgewählt aus der Gruppe bestehend aus F, Cl, CF₃, OCF₃, Alkyl mit 1, 2 oder 3 C-Atomen oder Alkoxy mit 1 oder 2 C-Atomen;
R(4) Wasserstoff;
R(5), R(6) und R(7) unabhängig voneinander Wasserstoff, F, Cl, CF₃, CN, COOMe, CONH₂, COMe, NH₂, OH, Alkyl mit 1, 2 oder 3 C-Atomen, Alkoxy mit 1 oder 2 C-Atomen;
R(30) und R(31) unabhängig voneinander Wasserstoff oder Methyl;
sowie ihre pharmazeutisch akzeptablen Salze.

6. Verbindungen der Formel I a und I b nach Ansprüchen 1 - 5, in der bedeuten:
X Sauerstoff oder Schwefel;
R(1) C(O)OR(9) oder COR(11);
R(9) und R(11) unabhängig voneinander CₓH₂ₓ-R(14);
x 0, 1, 2 oder 3;
R(14) Cycloalkyl mit 5 oder 6 C-Atomen oder Phenyl,
wobei Phenyl unsubstituiert sind oder substituiert mit 1 oder 2 Substituenten ausgewählt aus der Gruppe bestehend aus F, Cl, Br, I, CF₃, OCF₃, OH, Alkyl mit 1, 2, 3 oder Alkoxy mit 1 oder 2 C-Atomen;
R(2) Wasserstoff;
R(3) C_{y}H_{2y}-R(16);
y 0, 1 oder 2;
R(16) Alkyl mit 1, 2 oder 3 C-Atomen, Cycloalkyl mit 5 oder 6 C-Atomen, Phenyl oder Pyridyl,
wobei Phenyl und Pyridyl unsubstituiert sind oder substituiert mit 1, 2 oder 3 Substituenten ausgewählt aus der Gruppe bestehend aus F, Cl, CF₃, Alkyl mit 1, 2 oder 3 C-Atomen oder Alkoxy mit 1 oder 2 C-Atomen;
R(4) Wasserstoff;
R(5), R(6) und R(7) unabhängig voneinander Wasserstoff, F, Cl, Alkyl mit 1, 2 oder 3 C-Atomen oder Alkoxy mit 1, 2 C-Atomen;
R(30) und R(31) Wasserstoff;
sowie ihre pharmazeutisch akzeptablen Salze.

7. Verbindungen der Formel I a und I b nach einem oder mehreren der Ansprüche 1 bis 6 und ihre pharmazeutisch akzeptablen Salze zur Anwendung als Arzneimittel.

8. Pharmazeutische Zubereitung, enthaltend eine wirksame Menge mindestens einer Verbindung der Formel I nach einem oder mehreren der Ansprüche 1 bis 6 und/oder eines pharmazeutisch akzeptablen Salzes davon als Wirkstoff, zusammen mit pharmazeutisch annehmbaren Träger- und Zusatzstoffen und gegebenenfalls noch einem oder mehreren anderen pharmakologischen Wirkstoffen.

9. Verwendung einer Verbindung der Formel I a und I b nach einem oder mehreren der Ansprüche 1 bis 6 und/oder eines pharmazeutisch akzeptablen Salzes davon zur Herstellung eines Medikaments mit K⁺-Kanal-blockierender Wirkung zur Therapie und Prophylaxe von K⁺-Kanal mediierten Krankheiten.

10. Verwendung einer Verbindung der Formel I a und I b nach einem oder mehreren der Ansprüche 1 bis 6 und/oder eines pharmazeutisch akzeptablen Salzes davon zur Herstellung eines Medikaments zur Therapie oder Prophylaxe von Herzrhythmusstörungen, die durch Aktionspotential-Verlängerung behoben werden können.

11. Verwendung einer Verbindung der Formel I a und I b nach einem oder mehreren der Ansprüche 1 bis 6 und/oder eines pharmazeutisch akzeptablen Salzes davon zur Herstellung eines Medikaments zur Therapie oder Prophylaxe von Reentry-Arrhythmien.

12. Verwendung einer Verbindung der Formel I a und I b nach einem oder mehreren der Ansprüche 1 bis 6 und/oder eines pharmazeutisch akzeptablen Salzes davon zur Herstellung eines Medikaments zur Therapie oder Prophylaxe von supraventrikulären Arrhythmien.

13. Verwendung einer Verbindung der Formel I a und I b nach einem oder mehreren der Ansprüche 1 bis 6 und/oder eines pharmazeutisch akzeptablen Salzes davon zur Herstellung eines Medikaments zur Therapie oder Prophylaxe von atrialer Fibrillation oder atrialem Flattern.

14. Verwendung einer Verbindung der Formel I a und I b nach einem oder mehreren der Ansprüche 1 bis 6 und/oder eines pharmazeutisch akzeptablen Salzes davon zur Herstellung eines Medikamentes zur Terminierung von atrialer Fibrillation oder atrialem Flattern (Kardioversion).

15. Pharmazeutische Zubereitung, enthaltend eine wirksame Menge mindestens einer Verbindung der Formel I a und I b nach einem oder mehreren der Ansprüche 1 bis 6 und/oder eines pharmazeutisch akzeptablen Salzes davon sowie eines IKᵣ-Kanalblockers als Wirkstoffe, zusammen mit pharmazeutisch annehmbaren Trägerund Zusatzstoffen.

16. Pharmazeutische Zubereitung, enthaltend eine wirksame Menge mindestens einer Verbindung der Formel I a und I b nach einem oder mehreren der Ansprüche 1 bis 6 und/oder eines pharmazeutisch akzeptablen Salzes davon sowie eines IKₛ-Kanalblockers als Wirkstoffe, zusammen mit pharmazeutisch annehmbaren Träger- und Zusatzstoffen.

17. Pharmazeutische Zubereitung, enthaltend eine wirksame Menge mindestens einer Verbindung der Formel I a und I b nach einem oder mehreren der Ansprüche 1 bis 6 und/oder eines pharmazeutisch akzeptablen Salzes davon sowie eines Beta-blockers als Wirkstoffe, zusammen mit pharmazeutisch annehmbaren Träger- und Zusatzstoffen.

## Revendications

1. Composés de formules Ia et Ib 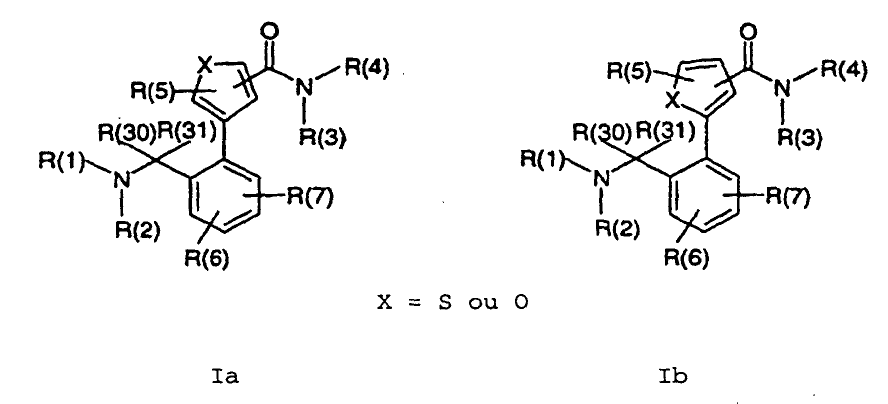 dans lesquelles :
X représente un atome d'oxygène ou de soufre ;
R(1) représente C(O)OR(9), SO₂R(10), COR(11), C(O)NR(12)R(13) ou C(S)NR(12)R(13) ;
R(9), R(10), R(11) et R(12) représentent, indépendamment les uns des autres, CₓH₂ₓ-R(14) ;
x représente 0, 1, 2, 3 ou 4,
x ne pouvant pas être 0 lorsque R(14) représente OR(15) ou SO₂Me ;
R(14) représente un groupe alkyle ayant 1, 2, 3, 4, 5 ou 6 atomes de carbone, cycloalkyle ayant 3, 4, 5, 6, 7, 8, 9, 10 ou 11 atomes de carbone, CF₃, C₂F₅, C₃F₇, CH₂F, CHF₂, OR(15), SO₂Me, phényle, naphtyle, biphénylyle, furyle, thiényle ou un groupe hétéroaromatique azoté à 1, 2, 3, 4, 5, 6, 7, 8 ou 9 atomes de carbone,
les groupes phényle, naphtyle, biphénylyle, furyle, thiényle et le groupe hétéroaromatique azoté étant non substitués ou portant 1, 2 ou 3 substituants choisis dans l'ensemble constitué par F, Cl, Br, I, CF₃, OCF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
R(15) représente un groupe alkyle ayant 1, 2, 3, 4 ou 5 atomes de carbone, cycloalkyle ayant 3, 4, 5 ou 6 atomes de carbone, CF₃ ou phényle, qui est non substitué ou porte 1, 2 ou 3 substituants choisis dans l'ensemble constitué par F, Cl, Br, I, CF₃, NO₂, CN, COOMe, CONH₂, COMe, NH₂, OH, des groupes alkyle ayant 1, 2, 3 ou 4 atomes de carbone, alcoxy ayant 1, 2, 3, ou 4 atomes de carbone, diméthylamino, sulfamoyle, méthylsulfonyle et méthylsulfonylamino ;
R(13) représente un atome d'hydrogène ou un groupe alkyle ayant 1, 2, 3 ou 4 atomes de carbone ou CF₃ ;
R(2) représente un atome d'hydrogène ou un groupe alkyle ayant 1, 2, 3 ou 4 atomes de carbone ou CF₃ ;
R(3) représente C_{y}H_{2y}-R(16) ;
y représente 0, 1, 2, 3 ou 4,
y ne pouvant pas être 0 lorsque R(16) représente OR(17) ou SO₂Me ;
R(16) représente un groupe alkyle ayant 1, 2, 3, 4, 5 ou 6 atomes de carbone, cycloalkyle ayant 3, 4, 5, 6, 7, 8, 9, 10 ou 11 atomes de carbone, CF₃, C₂F₅, C₃F₇, CH₂F, CHF₂, OR(17), SO₂Me, phényle, naphtyle, furyle, thiényle ou un groupe hétéroaromatique azoté à 1, 2, 3, 4, 5, 6, 7, 8 ou 9 atomes de carbone,
les groupes phényle, naphtyle, furyle, thiényle et le groupe hétéroaromatique azoté étant non substitués ou portant 1, 2 ou 3 substituants choisis dans l'ensemble constitué par F, Cl, Br, I, CF₃, OCF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
R(17) représente un atome d'hydrogène ou un groupe alkyle ayant 1, 2, 3, 4 ou 5 atomes de carbone, cycloalkyle ayant 3, 4, 5 ou 6 atomes de carbone, CF₃, phényle ou 2-, 3- ou 4-pyridyle ;
le groupe phényle ou le groupe 2-, 3- ou 4-pyridyle étant non substitué ou portant 1, 2 ou 3 substituants choisis dans l'ensemble constitué par F, Cl, Br, I, CF₃, OCF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
ou
R(3) représente CHR(18)R(19) ;
R(18) représente un atome d'hydrogène ou C_{z}H_{2z}-R(16), R(16) étant défini comme indiqué plus haut ;
z est 0, 1, 2 ou 3 ;
R(19) représente COOH, CONH₂, CONR(20)R(21), COOR(22) ou CH₂OH ;
R(20) représente un atome d'hydrogène ou un groupe alkyle ayant 1, 2, 3, 4 ou 5 atomes de carbone, CᵥH₂ᵥ-CF₃ ou C_{w}H_{2w}-phényle,
le cycle phényle étant non substitué ou portant 1, 2 ou 3 substituants choisis dans l'ensemble constitué par F, Cl, Br, I, CF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
v représente 0, 1, 2 ou 3 ;
w représente 0, 1, 2 ou 3 ;
R(21) représente un atome d'hydrogène ou un groupe alkyle ayant 1, 2, 3, 4 ou 5 atomes de carbone ;
R(22) représente un groupe alkyle ayant 1, 2, 3, 4 ou 5 atomes de carbone ;
R(4) représente un atome d'hydrogène ou un groupe alkyle ayant 1, 2, 3, 4, 5 ou 6 atomes de carbone ou CF₃ ;
ou
R(3) et R(4) forment ensemble une chaîne de 4 ou 5 groupes méthylène, dont un groupe méthylène peut être remplacé par -O-, -S-, -NH-, -N(méthyle)- ou -N(benzyle)- ;
R(5), R(6) et R(7) représentent, indépendamment les uns des autres, un atome d'hydrogène, F, Cl, Br, I, CF₃, NO₂, CN, COOMe, CONH₂, COMe, NH₂, OH, un groupe alkyle ayant 1, 2, 3 ou 4 atomes de carbone, alcoxy ayant 1, 2, 3 ou 4 atomes de carbone, diméthylamino, sulfamoyle, méthylsulfonyle ou méthylsulfonylamino ;
R(30) et R(31) représentent, indépendamment l'un de l'autre, un atome d'hydrogène ou un groupe alkyle ayant 1, 2 ou 3 atomes de carbone ;
ou
R(30) et R(31) représentent ensemble un atome d'oxygène ou une chaîne de 2 groupes méthylène,
et sels pharmaceutiquement acceptables de tels composés.

2. Composés de formules Ia et Ib selon la revendication 1, dans lesquels :
X représente un atome d'oxygène ou de soufre ;
R(1) représente C(O)OR(9), SO₂R(10), COR(11) ou C(O)NR(12)R(13) ;
R(9), R(10), R(11) et R(12) représentent, indépendamment les uns des autres, CₓH₂ₓ-R(14) ;
x représente 0, 1, 2, 3 ou 4,
x ne pouvant pas être 0 lorsque R(14) représente OR(15) ;
R(14) représente un groupe alkyle ayant 1, 2, 3, 4 atomes de carbone, cycloalkyle ayant 3, 4, 5, 6, 7, 8, 9 atomes de carbone, CF₃, OR(15), phényle, naphtyle, biphénylyle, furyle, thiényle ou un groupe hétéroaromatique azoté à 1, 2, 3, 4, 5, 6, 7, 8 ou 9 atomes de carbone,
les groupes phényle, naphtyle, biphénylyle, furyle, thiényle et le groupe hétéroaromatique azoté étant non substitués ou portant 1, 2 ou 3 substituants choisis dans l'ensemble constitué par F, Cl, Br, I, CF₃, OCF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
R(15) représente un groupe alkyle ayant 1, 2, 3, 4 ou 5 atomes de carbone, cycloalkyle ayant 3, 4, 5 ou 6 atomes de carbone, CF₃ ou phényle, qui n'est pas substitué ou porte 1, 2 ou 3 substituants choisis dans l'ensemble constitué par F, Cl, Br, I, CF₃, NO₂, CN, COOMe, CONH₂, COMe, NH₂, OH, des groupes alkyle ayant 1, 2, 3 ou 4 atomes de carbone, alcoxy ayant 1, 2, 3, ou 4 atomes de carbone, diméthylamino, sulfamoyle, méthylsulfonyle et méthylsulfonylamino ;
R(13) représente un atome d'hydrogène ou un groupe alkyle ayant 1, 2, 3 ou 4 atomes de carbone ou CF₃ ;
R(2) représente un atome d'hydrogène ou un groupe alkyle ayant 1, 2, 3 ou 4 atomes de carbone ou CF₃ ;
R(3) représente C_{y}H_{2y}-R(16) ;
y représente 0, 1, 2, 3 ou 4,
y ne pouvant pas être 0 lorsque R(16) représente OR(17) ou SO₂Me ;
R(16) représente un groupe alkyle ayant 1, 2, 3, 4, 5 ou 6 atomes de carbone, cycloalkyle ayant 3, 4, 5, 6, 7, 8, 9 atomes de carbone, CF₃, OR(17), SO₂Me, phényle, naphtyle, furyle, thiényle ou un groupe hétéroaromatique azoté à 1, 2, 3, 4, 5, 6, 7, 8 ou 9 atomes de carbone,
les groupes phényle, naphtyle, furyle, thiényle et le groupe hétéroaromatique azoté étant non substitués ou portant 1, 2 ou 3 substituants choisis dans l'ensemble constitué par F, Cl, Br, I, CF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
R(17) représente un atome d'hydrogène ou un groupe alkyle ayant 1, 2, 3, 4 ou 5 atomes de carbone, cycloalkyle ayant 3, 4, 5 ou 6 atomes de carbone, CF₃, phényle ou 2-, 3- ou 4-pyridyle ;
le groupe phényle ou le groupe 2-, 3- ou 4-pyridyle étant non substitués ou portant 1, 2 ou 3 substituants choisis dans l'ensemble constitué par F, Cl, Br, I, CF₃, OCF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
ou
R(3) représente CHR(18)R(19) ;
R(18) représente un atome d'hydrogène ou C_{z}H_{2z}-R(16), R(16) étant défini comme indiqué plus haut ;
z est 0, 1, 2 ou 3 ;
R(19) représente CONH₂, CONR(20)R(21), COOR(22) ou CH₂OH ;
R(20) représente un atome d'hydrogène ou un groupe alkyle ayant 1, 2, 3, 4 ou 5 atomes de carbone, CᵥH₂ᵥ-CF₃ ou C_{w}H_{2w}-phényle,
le cycle phényle étant non substitué ou portant 1, 2 ou 3 substituants choisis dans l'ensemble constitué par F, Cl, Br, I, CF₃, OCF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
v représente 0, 1, 2 ou 3 ;
w représente 0, 1, 2 ou 3 ;
R(21) représente un atome d'hydrogène ou un groupe alkyle ayant 1, 2, 3, 4 ou 5 atomes de carbone ;
R(22) représente un groupe alkyle ayant 1, 2, 3, 4 ou 5 atomes de carbone ;
R(4) représente un atome d'hydrogène ou un groupe alkyle ayant 1, 2, 3, 4, 5 ou 6 atomes de carbone ou CF₃ ;
R(5), R(6) et R(7) représentent, indépendamment les uns des autres, un atome d'hydrogène, F, Cl, Br, I, CF₃, NO₂, CN, COOMe, CONH₂, COMe, NH₂, OH, un groupe alkyle ayant 1, 2, 3 ou 4 atomes de carbone, alcoxy ayant 1, 2, 3 ou 4 atomes de carbone, diméthylamino, sulfamoyle, méthylsulfonyle ou méthylsulfonylamino ;
R(30) et R(31) représentent, indépendamment l'un de l'autre, un atome d'hydrogène ou un groupe alkyle ayant 1, 2 ou 3 atomes de carbone ;
ou
R(30) et R(31) représentent une chaîne de 2 groupes méthylène,
et sels pharmaceutiquement acceptables de tels composés.

3. Composés de formules Ia et Ib selon les revendications 1 et 2, dans lesquels :
X représente un atome d'oxygène ou de soufre ;
R(1) représente C(O)OR(9), SO₂R(10), COR(11) ou C(O)NR(12)R(13) ;
R(9), R(10), R(11) et R(12) représentent, indépendamment les uns des autres, CₓH₂ₓ-R(14) ;
x représente 0, 1, 2, 3 ou 4,
x ne pouvant pas être 0 lorsque R(14) représente OR(15) ;
R(14) représente un groupe alkyle ayant 1, 2, 3, 4, 5 ou 6 atomes de carbone, cycloalkyle ayant 3, 4, 5, 6, 7, 8, 9, 10 ou 11 atomes de carbone, CF₃, C₂F₅, C₃F₇, CH₂F, CHF₂, OR(15), phényle, naphtyle, biphénylyle, furyle, thiényle ou un groupe hétéroaromatique azoté à 1, 2, 3, 4, 5, 6, 7, 8 ou 9 atomes de carbone,
les groupes phényle, naphtyle, biphénylyle, furyle, thiényle et le groupe hétéroaromatique azoté étant non substitués ou portant 1, 2 ou 3 substituants choisis dans l'ensemble constitué par F, Cl, Br, I, CF₃, OCF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
R(15) représente un groupe alkyle ayant 1, 2, 3, 4 ou 5 atomes de carbone, cycloalkyle ayant 3, 4, 5 ou 6 atomes de carbone, CF₃ ou phényle, qui n'est pas substitué ou porte 1, 2 ou 3 substituants choisis dans l'ensemble constitué par F, Cl, Br, I, CF₃, NO₂, CN, COOMe, CONH₂, COMe, NH₂, OH, des groupes alkyle ayant 1, 2, 3 ou 4 atomes de carbone, alcoxy ayant 1, 2, 3, ou 4 atomes de carbone, diméthylamino, sulfamoyle, méthylsulfonyle et méthylsulfonylamino ;
R(13) représente un atome d'hydrogène ;
R(2) représente un atome d'hydrogène ou un groupe alkyle ayant 1, 2, 3 atomes de carbone ;
R(3) représente CHR(18)R(19) ;
R(18) représente un atome d'hydrogène ou C_{z}H_{2z}-R(16) ;
R(16) représente un groupe alkyle ayant 1, 2, 3, 4, 5 ou 6 atomes de carbone, cycloalkyle ayant 3, 4, 5, 6, 7, 8, 9 atomes de carbone, CF₃, OR(17), SO₂Me, phényle, naphtyle, furyle, thiényle ou un groupe hétéroaromatique azoté à 1, 2, 3, 4, 5, 6, 7, 8 ou 9 atomes de carbone,
les groupes phényle, naphtyle, furyle, thiényle et le groupe hétéroaromatique azoté étant non substitués ou portant 1, 2 ou 3 substituants choisis dans l'ensemble constitué par F, Cl, Br, I, CF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
R(17) représente un atome d'hydrogène ou un groupe alkyle ayant 1, 2, 3, 4 ou 5 atomes de carbone, cycloalkyle ayant 3, 4, 5 ou 6 atomes de carbone, CF₃, phényle ou 2-, 3- ou 4-pyridyle ;
le groupe phényle ou le groupe 2-, 3- ou 4-pyridyle étant non substitués ou portant 1, 2 ou 3 substituants choisis dans l'ensemble constitué par F, Cl, Br, I, CF₃, OCF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
z représente 0, 1, 2 ou 3 ;
R(19) représente CONH₂, CONR(20)R(21), COOR(22) ou CH₂OH ;
R(20) représente un atome d'hydrogène ou un groupe alkyle ayant 1, 2, 3, 4 ou 5 atomes de carbone, CᵥH₂ᵥ-CF₃ ou C_{w}H_{2w}-phényle,
le cycle phényle étant non substitué ou portant 1, 2 ou 3 substituants choisis dans l'ensemble constitué par F, Cl, Br, I, CF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
v représente 0, 1, 2 ou 3 ;
w représente 0, 1, 2 ou 3 ;
R(21) représente un atome d'hydrogène ou un groupe alkyle ayant 1, 2, 3, 4 ou 5 atomes de carbone ;
R(22) représente un groupe alkyle ayant 1, 2, 3, 4 ou 5 atomes de carbone ;
R(4) représente un atome d'hydrogène ou un groupe alkyle ayant 1 ou 2 atomes de carbone ;
R(5), R(6) et R(7) représentent, indépendamment les uns des autres, un atome d'hydrogène, F, Cl, Br, I, CF₃, NO₂, CN, COOMe, CONH₂, COMe, NH₂, OH, un groupe alkyle ayant 1, 2, 3 ou 4 atomes de carbone, alcoxy ayant 1, 2, 3 ou 4 atomes de carbone, diméthylamino, sulfamoyle, méthylsulfonyle ou méthylsulfonylamino ;
R(30) et R(31) représentent, indépendamment l'un de l'autre, un atome d'hydrogène ou le groupe méthyle ;
et sels pharmaceutiquement acceptables de tels composés.

4. Composés de formules Ia et Ib selon les revendications 1 à 3, dans lesquels :
X représente un atome d'oxygène ou de soufre ;
R(1) représente C(O)OR(9), SO₂R(10), COR(11) ou C(O)NR(12)R(13) ;
R(9), R(10), R(11) et R(12) représentent, indépendamment les uns des autres, CₓH₂ₓ-R(14) ;
x représente 0, 1, 2, 3 ou 4 ;
R(14) représente un groupe alkyle ayant 1, 2, 3, 4, 5 ou 6 atomes de carbone, cycloalkyle ayant 3, 4, 5, 6, 7, 8, 9, 10 ou 11 atomes de carbone, CF₃, C₂F₅, C₃F₇, CH₂F, CHF₂, SO₂Me, phényle, naphtyle, biphénylyle, furyle, thiényle ou un groupe hétéroaromatique azoté à 1, 2, 3, 4, 5, 6, 7, 8 ou 9 atomes de carbone,
les groupes phényle, naphtyle, biphénylyle, furyle, thiényle et le groupe hétéroaromatique azoté étant non substitués ou portant 1, 2 ou 3 substituants choisis dans l'ensemble constitué par F, Cl, Br, I, CF₃, OCF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
R(13) représente un atome d'hydrogène ;
R(2) représente un atome d'hydrogène ou le groupe méthyle ;
R(3) représente C_{y}H_{2y}-R(16) ;
y représente 0, 1, 2, 3 ou 4,
y ne pouvant pas être 0 lorsque R(16) représente OR(17) ;
R(16) représente un groupe alkyle ayant 1, 2, 3, 4, 5 ou 6 atomes de carbone, cycloalkyle ayant 3, 4, 5, 6, 7, 8, 9, 10 ou 11 atomes de carbone, CF₃, C₂F₅, C₃F₇, CH₂F, CHF₂, OR(17), SO₂Me, phényle, naphtyle, furyle, thiényle ou un groupe hétéroaromatique azoté à 1, 2, 3, 4, 5, 6, 7, 8 ou 9 atomes de carbone,
les groupes phényle, naphtyle, furyle, thiényle et le groupe hétéroaromatique azoté étant non substitués ou portant 1, 2 ou 3 substituants choisis dans l'ensemble constitué par F, Cl, Br, I, CF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
R(17) représente un atome d'hydrogène ou un groupe alkyle ayant 1, 2, 3, 4 ou 5 atomes de carbone, cycloalkyle ayant 3, 4, 5 ou 6 atomes de carbone, CF₃, phényle ou 2-, 3- ou 4-pyridyle ;
le groupe phényle ou 2-, 3- ou 4-pyridyle étant non substitué ou portant 1, 2 ou 3 substituants choisis dans l'ensemble constitué par F, Cl, Br, I, CF₃, NO₂, CN, COOMe, CONH₂, COMe, NH₂, OH, des groupes alkyle ayant 1, 2, 3 ou 4 atomes de carbone, alcoxy ayant 1, 2, 3 ou 4 atomes de carbone, diméthylamino, sulfamoyle, méthylsulfonyle et méthylsulfonylamino ;
R(4) représente un atome d'hydrogène ou un groupe alkyle ayant 1 ou 2 atomes de carbone ;
R(5), R(6) et R(7) représentent, indépendamment les uns des autres, un atome d'hydrogène, F, Cl, Br, I, CF₃, NO₂, CN, COOMe, CONH₂, COMe, NH₂, OH, un groupe alkyle ayant 1, 2, 3 ou 4 atomes de carbone, alcoxy ayant 1, 2, 3 ou 4 atomes de carbone, diméthylamino, sulfamoyle, méthylsulfonyle ou méthylsulfonylamino ;
R(30) et R(31) représentent, indépendamment l'un de l'autre, un atome d'hydrogène ou le groupe méthyle ;
et sels pharmaceutiquement acceptables de tels composés.

5. Composés de formule Ia ou Ib selon les revendications 1 à 4, dans lesquels :
R(1) représente C(O)OR(9), SO₂R(10), COR(11) ou C(O)NR(12)R(13) ;
R(9), R(10), R(11) et R(12) représentent, indépendamment les uns des autres, CₓH₂ₓ-R(14) ;
x représente 0, 1, 2 ou 3 ;
R(14) représente un groupe alkyle ayant 1, 2, 3 ou 4 atomes de carbone, cycloalkyle ayant 3, 4, 5, 6, 7, 8 ou 9 atomes de carbone, CF₃, phényle ou pyridyle,
les groupes phényle et pyridyle étant non substitués ou portant 1 ou 2 substituants choisis dans l'ensemble constitué par F, Cl, Br, I, CF₃, OCF₃, OH, des groupes alkyle ayant 1, 2 ou 3 atomes de carbone ou alcoxy ayant 1 ou 2 atomes de carbone ;
R(13) représente un atome d'hydrogène ;
R(2) représente un atome d'hydrogène ;
R(3) représente C_{y}H_{2y}-R(16) ;
y représente 0, 1 ou 2 ;
R(16) représente un groupe alkyle ayant 1, 2 ou 3 atomes de carbone, cycloalkyle ayant 5 ou 6 atomes de carbone, CF₃, phényle ou pyridyle,
les groupes phényle et pyridyle étant non substitués ou portant 1 ou 2 substituants choisis dans l'ensemble constitué par F, Cl, CF₃, OCF₃, des groupes alkyle ayant 1, 2 ou 3 atomes de carbone et alcoxy ayant 1 ou 2 atomes de carbone ;
R(4) représente un atome d'hydrogène ;
R(5), R(6) et R(7) représentent, indépendamment les uns des autres, un atome d'hydrogène, F, Cl, CF₃, CN, COOMe, CONH₂, COMe, NH₂, OH, des groupes alkyle ayant 1, 2 ou 3 atomes de carbone, alcoxy ayant 1 ou 2 atomes de carbone ;
R(30) et R(31) représentent, indépendamment l'un de l'autre, un atome d'hydrogène ou le groupe méthyle ;
et sels physiologiquement acceptables de tels composés.

6. Composés de formules Ia et Ib selon les revendications 1 à 5, dans lesquels :
X représente un atome d'oxygène ou de soufre ;
R(1) représente C(O)OR(9) ou COR(11) ;
R(9) et R(11) représentent, indépendamment l'un de l'autre, CₓH₂ₓ-R(14) ;
x représente 0, 1, 2 ou 3 ;
R(14) représente un groupe cycloalkyle ayant 5 ou 6 atomes de carbone ou phényle,
le groupe phényle étant non substitué ou portant 1 ou 2 substituants choisis dans l'ensemble constitué par F, Cl, Br, I, CF₃, OCF₃, OH, des groupes alkyle ayant 1, 2, 3 atomes de carbone ou alcoxy ayant 1 ou 2 atomes de carbone ;
R(2) représente un atome d'hydrogène ;
R(3) représente un groupe C_{y}H_{2y}-R(16) ;
y représente 0, 1 ou 2 ;
R(16) représente un groupe alkyle ayant 1, 2 ou 3 atomes de carbone, cycloalkyle ayant 5 ou 6 atomes de carbone, phényle ou pyridyle,
les groupes phényle et pyridyle étant non substitués ou portant 1, 2 ou 3 substituants choisis dans l'ensemble constitué par F, Cl, CF₃, des groupes alkyle ayant 1, 2 ou 3 atomes de carbone ou alcoxy ayant 1 ou 2 atomes de carbone ;
R(4) représente un atome d'hydrogène ;
R(5), R(6) et R(7) représentent, indépendamment les uns des autres, un atome d'hydrogène, F, Cl ou un groupe alkyle ayant 1, 2 ou 3 atomes de carbone ou alcoxy ayant 1 ou 2 atomes de carbone ;
R(30) et R(31) représentent un atome d'hydrogène.
et sels pharmaceutiquement acceptables de tels composés.

7. Composés de formules Ia et Ib selon une ou plusieurs des revendications 1 à 6 et sels pharmaceutiquement acceptables de tels composés, pour utilisation en tant que médicament.

8. Préparation pharmaceutique contenant une quantité efficace d'au moins un composé de formule I selon une ou plusieurs des revendications 1 à 6 et/ou d'un sel pharmaceutique d'un tel composé, en tant que substance active, conjointement avec des véhicules et additifs pharmaceutiquement acceptables et éventuellement encore une ou plusieurs autres substances actives pharmacologiques.

9. Utilisation d'un composé de formules Ia et Ib selon une ou plusieurs des revendications 1 à 6 et/ou d'un sel pharmaceutiquement acceptable d'un tel composé, pour la fabrication d'un médicament à action bloquant le canal K⁺, pour le traitement et la prophylaxie de maladies médiées par le canal K⁺.

10. Utilisation d'un composé de formules Ia et Ib selon une ou plusieurs des revendications 1 à 6 et/ou d'un sel pharmaceutiquement acceptable d'un tel composé, pour la fabrication d'un médicament destiné au traitement ou à la prophylaxie de troubles du rythme cardiaque, qui peuvent être supprimés par prolongement du potentiel d'action.

11. Utilisation d'un composé de formules la et Ib selon une ou plusieurs des revendications 1 à 6 et/ou d'un sel pharmaceutiquement acceptable d'un tel composé, pour la fabrication d'un médicament destiné au traitement ou à la prophylaxie d'arythmies de ré-entrée.

12. Utilisation d'un composé de formules Ia et Ib selon une ou plusieurs des revendications 1 à 6 et/ou d'un sel pharmaceutiquement acceptable d'un tel composé, pour la fabrication d'un médicament destiné au traitement ou à la prophylaxie d'arythmies supraventriculaires.

13. Utilisation d'un composé de formules Ia et Ib selon une ou plusieurs des revendications 1 à 6 et/ou d'un sel pharmaceutiquement acceptable d'un tel composé, pour la fabrication d'un médicament destiné au traitement ou à la prophylaxie de la fibrillation auriculaire ou du flutter auriculaire.

14. Utilisation d'un composé de formules Ia et Ib selon une ou plusieurs des revendications 1 à 6 et/ou d'un sel pharmaceutiquement acceptable d'un tel composé, pour la fabrication d'un médicament destiné à la terminaison de la fibrillation auriculaire ou du flutter auriculaire (cardioversion).

15. Préparation pharmaceutique contenant une quantité efficace d'au moins un composé de formules Ia et Ib selon une ou plusieurs des revendications 1 à 6 et/ou d'un sel pharmaceutiquement acceptable d'un tel composé ainsi que d'un inhibiteur de canal IKᵣ, en tant que substances actives, conjointement avec des véhicules et adjuvants pharmaceutiquement acceptables.

16. Préparation pharmaceutique contenant une quantité efficace d'au moins un composé de formules Ia et Ib selon une ou plusieurs des revendications 1 à 6 et/ou d'un sel pharmaceutiquement acceptable d'un tel composé, ainsi que d'un inhibiteur de canal IKₛ₋, en tant que substances actives, conjointement avec des véhicules et adjuvants pharmaceutiquement acceptables.

17. Préparation pharmaceutique contenant une quantité efficace d'au moins un composé de formules Ia et Ib selon une ou plusieurs des revendications 1 à 6 et/ou d'un sel pharmaceutiquement acceptable d'un tel composé, ainsi que d'un β-bloquant, en tant que substances actives, conjointement avec des véhicules et adjuvants pharmaceutiquement acceptables.

## Claims

1. A compound of the formula I a or I b, 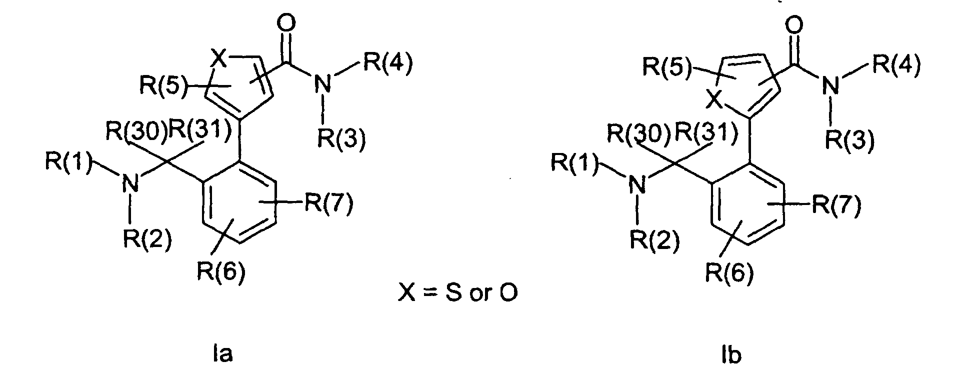 in which:
X is oxygen or sulfur;
R(1) is C(O)OR(9), SO₂R(10), COR(11), C(O)NR(12)R(13) or C(S)NR(12)R(13);
R(9), R(10), R(11) and R(12) independently of one another are CₓH₂ₓ-R(14);
x is 0, 1, 2, 3 or 4,
where x cannot be 0 if R(14) is OR(15) or SO₂Me;
R(14) is alkyl having 1, 2, 3, 4, 5 or 6 carbon atoms, cycloalkyl having 3, 4, 5, 6, 7, 8, 9, 10 or 11 carbon atoms, CF₃, C₂F₅, C₃F₇, CH₂F, CHF₂, OR(15), SO₂Me, phenyl, naphthyl, biphenylyl, furyl, thienyl or an N-containing heteroaromatic having 1, 2, 3, 4, 5, 6, 7, 8 or 9 carbon atoms,
where phenyl, naphthyl, biphenylyl, furyl, thienyl and the N-containing heteroaromatic are unsubstituted or substituted by 1, 2 or 3 substitutents selected from the group consisting of F, Cl, Br, I, CF₃, OCF₃, NO₂, CN, COOMe, CONH₂, COME, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(15) is alkyl having 1, 2, 3, 4 or 5 carbon atoms, cycloalkyl having 3, 4, 5 or 6 carbon atoms, CF₃ or phenyl, which is unsubstituted or substituted by 1, 2 or 3 substitutents selected from the group consisting of F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(13) is hydrogen, alkyl having 1, 2, 3 or 4 carbon atoms or CF₃;
R(2) is hydrogen, alkyl having 1, 2, 3 or 4 carbon atoms or CF₃;
R(3) is C_{y}H_{2y}-R(16);
y is 0, 1, 2, 3 or 4,
where y cannot be 0 if R(16) is OR(17) or SO₂Me;
R(16) is alkyl having 1, 2, 3, 4, 5 or 6 carbon atoms, cycloalkyl having 3, 4, 5, 6, 7, 8, 9, 10 or 11 carbon atoms, CF₃, C₂F₅, C₃F₇, CH₂F, CHF₂, OR(17), SO₂Me, phenyl, naphthyl, furyl, thienyl or an N-containing heteroaromatic having 1, 2, 3, 4, 5, 6, 7, 8 or 9 carbon atoms,
where phenyl, naphthyl, furyl, thienyl and the N-containing heteroaromatic are unsubstituted or substituted by 1, 2 or 3 substitutents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino.
R(17) is hydrogen, alkyl having 1, 2, 3, 4 or 5 carbon atoms, cycloalkyl having 3, 4, 5 or 6 carbon atoms, CF₃, phenyl or 2-, 3- or 4- pyridyl,
where phenyl or 2-, 3- or 4- pyridyl is unsubstituted or substituted by 1, 2 or 3 substitutents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
or
R(3) is CHR(18)R(19);
R(18) is hydrogen or C_{z}H_{2z}-R(16), where R(16) is defined as indicated above;
z is 0, 1, 2 or 3;
R(19) is COOH, CONH₂, CONR(20)R(21), COOR(22) or CH₂OH;
R(20) is hydrogen, alkyl having 1, 2, 3, 4 or 5 carbon atoms, CᵥH₂ᵥ-CF₃ or C_{w}H_{2w}-phenyl,
where the phenyl ring is unsubstituted or substituted by 1, 2 or 3 substitutents selected from the group consisting of F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
v is 0, 1, 2 or 3;
w is 0, 1, 2 or 3;
R(21) is hydrogen or alkyl having 1, 2, 3, 4 or 5 carbon atoms;
R(22) is alkyl having 1, 2, 3, 4 or 5 carbon atoms;
R(4) is hydrogen, alkyl having 1, 2, 3, 4, 5 or 6 carbon atoms or CF₃;
or
R(3) and R(4) together are a chain of 4 or 5 methylene groups, of which one methylene group can be replaced by -O-, -S-, -NH-, -N(methyl)- or -N(benzyl)-;
R(5), R(6) and R(7) independently of one another are hydrogen, F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl or methylsulfonylamino.
R(30) and R(31) independently of one another are hydrogen or alkyl having 1, 2 or 3 carbon atoms;
or
R(30) and R(31) together are oxygen or a chain of 2 methylene groups;
or its pharmaceutically acceptable salts.

2. A compound of the formula I a or I b as claimed in claim 1, in which:
X is oxygen or sulfur;
R(1) is C(O)OR(9), SO₂R(10), COR(11) or C(O)NR(12)R(13);
R(9), R(10), R(11) and R(12) independently of one another are CₓH₂ₓ-R(14);
x is 0, 1, 2, 3 or 4,
where x cannot be 0 if R(14) is OR(15)
R(14) is alkyl having 1, 2, 3, 4 carbon atoms, cycloalkyl having 3, 4, 5, 6, 7, 8, 9 carbon atoms, CF₃, OR(15), phenyl, naphthyl, biphenylyl, furyl, thienyl or an N-containing heteroaromatic having 1, 2, 3, 4, 5, 6, 7, 8 or 9 carbon atoms,
where phenyl, naphthyl, biphenylyl, furyl, thienyl and the N-containing heteroaromatic are unsubstituted or substituted by 1, 2 or 3 substitutents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(15) is alkyl having 1, 2, 3, 4 or 5 carbon atoms, cycloalkyl having 3, 4, 5 or 6 carbon atoms, CF₃ or phenyl, which is unsubstituted or substituted by 1, 2 or 3 substitutents selected from the group consisting of F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(13) is hydrogen, alkyl having 1, 2, 3 or 4 carbon atoms or CF₃;
R(2) is hydrogen, alkyl having 1, 2, 3 or 4 carbon atoms or CF₃;
R(3) is C_{y}H_{2y}-R(16);
y is 0, 1, 2, 3 or 4,
where y cannot be 0 if R(16) is OR(17) or SO₂Me;
R(16) is alkyl having 1, 2, 3, 4, 5 or 6 carbon atoms, cycloalkyl having 3, 4, 5, 6, 7, 8 or 9 carbon atoms, CF₃, OR(17), SO₂Me, phenyl, naphthyl, furyl, thienyl or an N-containing heteroaromatic having 1, 2, 3, 4, 5, 6, 7, 8 or 9 carbon atoms,
where phenyl, naphthyl, furyl, thienyl and the N-containing heteroaromatic are unsubstituted or substituted by 1, 2 or 3 substitutents selected from the group consisting of F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(17) is hydrogen, alkyl having 1, 2, 3, 4 or 5 carbon atoms, cycloalkyl having 3, 4, 5 or 6 carbon atoms, CF₃, phenyl or 2-, 3- or 4- pyridyl,
where phenyl or 2-, 3- or 4- pyridyl are unsubstituted or substituted by 1, 2 or 3 substitutents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
or
R(3) is CHR(18)R(19);
R(18) is hydrogen or C_{z}H_{2z}-R(16), where R(16) is defined as indicated above;
z is 0, 1, 2 or 3;
R(19) is CONH₂, CONR(20)R(21), COOR(22) or CH₂OH;
R(20) is hydrogen, alkyl having 1, 2, 3, 4 or 5 carbon atoms, CᵥH₂ᵥ-CF₃ or C_{w}H_{2w}-phenyl,
where the phenyl ring is unsubstituted or substituted by 1, 2 or 3 substitutents selected from the group consisting of F, Cl, Br, I, CF₃, OCF₃, NO₂, CN, COOMe, CONH₂, COME, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
v is 0, 1, 2 or 3;
w is 0, 1, 2 or 3;
R(21) is hydrogen or alkyl having 1, 2, 3, 4 or 5 carbon atoms;
R(22) is alkyl having 1, 2, 3, 4 or 5 carbon atoms;
R(4) is hydrogen, alkyl having 1, 2, 3, 4, 5 or 6 carbon atoms or CF₃;
R(5), R(6) and R(7) independently of one another are hydrogen, F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl or methylsulfonylamino;
R(30) and R(31) independently of one another are hydrogen or alkyl having 1, 2 or 3 carbon atoms;
or
R(30) and R(31) are a chain of 2 methylene groups
or its pharmaceutically acceptable salts.

3. A compound of the formula I a or I b as claimed in claims 1 and 2 in which:
X is oxygen or sulfur;
R(1) is C(O)OR(9), SO₂R(10), COR(11) or C(O)NR(12)R(13);
R(9), R(10), R(11) and R(12) independently of one another are CₓH₂ₓ-R(14);
x is 0, 1, 2, 3 or 4,
where x cannot be 0 if R(14) is OR(15);
R(14) is alkyl having 1, 2, 3, 4, 5 or 6 carbon atoms, cycloalkyl having 3, 4, 5, 6, 7, 8, 9, 10 or 11 carbon atoms, CF₃, C₂F₅, C₃F₇, CH₂F, CHF₂, OR(15), phenyl, naphthyl, biphenylyl, furyl, thienyl or an N-containing heteroaromatic having 1, 2, 3, 4, 5, 6, 7, 8 or 9 carbon atoms,
where phenyl, naphthyl, biphenylyl, furyl, thienyl and the N-containing heteroaromatic are unsubstituted or substituted by 1, 2 or 3 substitutents selected from the group consisting of F, Cl, Br, I, CF₃, OCF₃, NO₂, CN, COOMe, CONH₂, COME, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(15) is alkyl having 1, 2, 3, 4 or 5 carbon atoms, cycloalkyl having 3, 4, 5 or 6 carbon atoms, CF₃ or phenyl, which is unsubstituted or substituted by 1, 2 or 3 substitutents selected from the group consisting of F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(13) is hydrogen;
R(2) is hydrogen or alkyl having 1, 2 or 3 carbon atoms;
R(3) is CHR(18)R(19);
R(18) is hydrogen or C_{z}H_{2z}-R(16),
R(16) is alkyl having 1, 2, 3, 4, 5 or 6 carbon atoms, cycloalkyl having 3, 4, 5, 6, 7, 8 or 9 carbon atoms, CF₃, OR(17), SO₂Me, phenyl, naphthyl, furyl, thienyl or an N-containing heteroaromatic having 1, 2, 3, 4, 5, 6, 7, 8 or 9 carbon atoms,
where phenyl, naphthyl, furyl, thienyl and the N-containing heteroaromatic are unsubstituted or substituted by 1, 2 or 3 substitutents selected from the group consisting of F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(17) is hydrogen, alkyl having 1, 2, 3, 4 or 5 carbon atoms, cycloalkyl having 3, 4, 5 or 6 carbon atoms, CF₃, phenyl or 2-, 3- or 4- pyridyl,
where phenyl or 2-, 3- or 4- pyridyl are unsubstituted or substituted by 1, 2 or 3 substitutents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
z is 0, 1, 2 or 3;
R(19) is CONH₂, CONR(20)R(21), COOR(22) or CH₂OH;
R(20) is hydrogen, alkyl having 1, 2, 3, 4 or 5 carbon atoms, CᵥH₂ᵥ-CF₃ or C_{w}H_{2w}-phenyl,
where the phenyl ring is unsubstituted or substituted by 1, 2 or 3 substitutents selected from the group consisting of F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
v is 0, 1, 2 or 3;
w is 0, 1, 2 or 3;
R(21) is hydrogen or alkyl having 1, 2, 3, 4 or 5 carbon atoms;
R(22) is alkyl having 1, 2, 3, 4 or 5 carbon atoms;
R(4) is hydrogen, alkyl having 1 or 2 carbon atoms
R(5), R(6) and R(7) independently of one another are hydrogen, F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl or methylsulfonylamino;
R(30) and R(31) independently of one another are hydrogen or methyl;
or its pharmaceutically acceptable salts.

4. A compound of the formula I a or I b as claimed in claims 1 - 3, in which:
X is oxygen or sulfur;
R(1) is C(O)OR(9), SO₂R(10), COR(11) or C(O)NR(12)R(13);
R(9), R(10), R(11) and R(12) independently of one another are CₓH₂ₓ-R(14);
x is 0, 1, 2, 3 or 4,
R(14) is alkyl having 1, 2, 3, 4, 5 or 6 carbon atoms, cycloalkyl having 3, 4, 5, 6, 7, 8, 9, 10 or 11 carbon atoms, CF₃, C₂F₅, C₃F₇, CH₂F, CHF₂, SO₂Me, phenyl, naphthyl, biphenylyl, furyl, thienyl or an N-containing heteroaromatic having 1, 2, 3, 4, 5, 6, 7, 8 or 9 carbon atoms,
where phenyl, naphthyl, biphenylyl, furyl, thienyl and the N-containing heteroaromatic are unsubstituted or substituted by 1, 2 or 3 substitutents selected from the group consisting of F, Cl, Br, I, CF₃, OCF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(13) is hydrogen;
R(2) is hydrogen or methyl;
R(3) is C_{y}H_{2y}-R(16);
y is 0, 1, 2, 3 or 4;
where y cannot be 0 if R(16) is OR(17);
R(16) is alkyl having 1, 2, 3, 4, 5 or 6 carbon atoms, cycloalkyl having 3, 4, 5, 6, 7, 8, 9, 10 or 11 carbon atoms, CF₃, C₂F₅, C₃F₇, CH₂F, CHF₂, OR(17), SO₂Me, phenyl, naphthyl, furyl, thienyl or an N-containing heteroaromatic having 1, 2, 3, 4, 5, 6, 7, 8 or 9 carbon atoms, where phenyl, naphthyl, furyl, thienyl and the N-containing heteroaromatic are unsubstituted or substituted by 1, 2 or 3 substitutents selected from the group consisting of F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(17) is hydrogen, alkyl having 1, 2, 3, 4 or 5 carbon atoms, cycloalkyl having 3, 4, 5 or 6 carbon atoms, CF₃, phenyl or 2-, 3- or 4- pyridyl,
where phenyl or 2-, 3- or 4- pyridyl are unsubstituted or substituted by 1, 2 or 3 substitutents selected from the group consisting of F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl and methylsulfonylamino;
R(4) is hydrogen or alkyl having 1 or 2 carbon atoms;
R(5), R(6) and R(7) independently of one another are hydrogen, F, Cl, Br, I, CF₃, NO₂, CN, COOMe, CONH₂, COMe, NH₂, OH, alkyl having 1, 2, 3 or 4 carbon atoms, alkoxy having 1, 2, 3 or 4 carbon atoms, dimethylamino, sulfamoyl, methylsulfonyl or methylsulfonylamino;
R(30) and R(31) independently of one another are hydrogen or methyl;
or its pharmaceutically acceptable salts.

5. A compound of the formula I a or I b as claimed in claims 1 - 4, in which:
R(1) is C(O)OR(9), SO₂R(10), COR(11) or C(O)NR(12)R(13);
R(9), R(10), R(11) and R(12) independently of one another are CₓH₂ₓ-R(14);
x is 0, 1, 2 or 3;
R(14) is alkyl having 1, 2, 3 or 4 carbon atoms, cycloalkyl having 3, 4, 5, 6, 7, 8 or 9 carbon atoms, CF₃, phenyl or pyridyl,
where phenyl and pyridyl are unsubstituted or substituted by 1 or 2 substitutents selected from the group consisting of F, Cl, Br, I, CF₃, OCF₃, OH, alkyl having 1, 2, 3, alkoxy having 1 or 2 carbon atoms;
R(13) is hydrogen:
R(2) is hydrogen;
R(3) is C_{y}H_{2y}-R(16);
y is 0, 1 or 2;
R(16) is alkyl having 1, 2 or 3 carbon atoms, cycloalkyl having 5 or 6 carbon atoms, CF₃, phenyl or pyridyl,
where phenyl and pyridyl are unsubstituted or substituted by 1 or 2 substituents selected from the group consisting of F, Cl, CF₃, OCF₃, alkyl having 1, 2 or 3 carbon atoms or alkoxy having 1 or 2 carbon atoms;
R(4) is hydrogen;
R(5), R(6) and R(7) independently of one another are hydrogen, F, Cl, CF₃, CN, COOMe, CONH₂, COMe, NH₂, OH, alkyl having 1, 2 or 3 carbon atoms, alkoxy having 1 or 2 carbon atoms;
R(30) and R(31) independently of one another are hydrogen or methyl;
or its pharmaceutically acceptable salts.

6. A compound of the formula I a or I b as claimed in claims 1 - 5, in which:
X is oxygen or sulfur;
R(1) is C(O)OR(9) or COR(11);
R(9) and R(11) independently of one another are CₓH₂ₓ-R(14);
x is 0, 1, 2 or 3;
R(14) is cycloalkyl having 5 or 6 carbon atoms or phenyl,
where phenyl are unsubstituted or substituted by 1 or 2 substitutents selected from the group consisting of F, Cl, Br, I, CF₃, OCF₃, OH, alkyl having 1, 2, 3 or alkoxy having 1 or 2 carbon atoms;
R(2) is hydrogen;
R(3) is C_{y}H_{2y}-R(16);
y is 0, 1 or 2;
R(16) is alkyl having 1, 2 or 3 carbon atoms, cycloalkyl having 5 or 6 carbon atoms, phenyl or pyridyl,
where phenyl and pyridyl are unsubstituted or substituted by 1, 2 or 3 substituents selected from the group consisting of F, Cl, CF₃, alkyl having 1, 2 or 3 carbon atoms or alkoxy having 1 or 2 carbon atoms;
R(4) is hydrogen;
R(5), R(6) and R(7) independently of one another are hydrogen, F, Cl, alkyl having 1, 2 or 3 carbon atoms or alkoxy having 1 or 2 carbon atoms;
R(30) and R(31) are hydrogen;
or its pharmaceutically acceptable salts.

7. A compound of the formula I a or I b as claimed in one or more of claims 1 to 6 or its pharmaceutically acceptable salts for use as a pharmaceutical.

8. A pharmaceutical preparation, comprising an efficacious amount of at least one compound of the formula I as claimed in one or more of claims 1 to 6 and/or of a pharmaceutically acceptable salt thereof as active compound, together with pharmaceutically acceptable vehicles and additives and, if appropriate, additionally one or more other pharmacological active compounds.

9. The use of a compound of the formula I a or I b as claimed in one or more of claims 1 to 6 and/or of a pharmaceutically acceptable salt thereof for the production of a medicament having K⁺ channel-blocking action for the therapy and prophylaxis of K⁺ channel-mediated diseases.

10. The use of a compound of the formula I a or I b as claimed in one or more of claims 1 to 6 and/or of a pharmaceutically acceptable salt thereof for the production of a medicament for the therapy or prophylaxis of cardiac arrhythmias which can be eliminated by action potential prolongation.

11. The use of a compound of the formula I a or I b as claimed in one or more of claims 1 to 6 and/or of a pharmaceutically acceptable salt thereof for the production of a medicament for the therapy or prophylaxis of reentry arrhythmias.

12. The use of a compound of the formula I a or I b as claimed in one or more of claims 1 to 6 and/or of a pharmaceutically acceptable salt thereof for the production of a medicament for the therapy or prophylaxis of supraventricular arrhythmias.

13. The use of a compound of the formula I a or I b as claimed in one or more of claims 1 to 6 and/or of a pharmaceutically acceptable salt thereof for the production of a medicament for the therapy or prophylaxis of atrial fibrillation or atrial flutters.

14. The use of a compound of the formula I a or I b as claimed in one or more of claims 1 to 6 and/or of a pharmaceutically acceptable salt thereof for the production of a medicament for the termination of atrial fibrillation or atrial flutters (cardioversion).

15. A pharmaceutical preparation, comprising an efficacious amount of at least one compound of the formula I a or I b as claimed in one or more of claims 1 to 6 and/or of a pharmaceutically acceptable salt thereof and of an IKᵣ channel blocker as active compounds, together with pharmaceutically acceptable vehicles and additives.

16. A pharmaceutical preparation, comprising an efficacious amount of at least one compound of the formula I a or I b as claimed in one or more of claims 1 to 6 and/or of a pharmaceutically acceptable salt thereof and of an IKₛ channel blocker as active compounds, together with pharmaceutically acceptable vehicles and additives.

17. A pharmaceutical preparation, comprising an efficacious amount of at least one compound of the formula I a or I b as claimed in one or more of claims 1 to 6 and/or of a pharmaceutically acceptable salt thereof and of a beta blocker as active compounds, together with pharmaceutically acceptable vehicles and additives.
